# EUROPEAN PATENT APPLICATION

(11) **EP 4 353 226 A1**
(43) Date of publication of application: **17.04.2024**
(21) Application number: 22820562.1
(22) Date of filing: 08.06.2022
(51) Int. Cl.: A61K 31/352, A61P 37/00, A61P 27/02

(54) **PHARMACEUTICAL COMPOSITION FOR PREVENTING OR TREATING DEGENERATIVE EYE DISEASES**

(30) Priority: 08.06.2021 KR 20210074192
(71) Applicant: Glaceum, Inc., Gyeonggi-do 16675 (KR)
(72) Inventor: YOO, Sang Ku, Suwon-si Gyeonggi-do 16223 (KR); PARK, Hyung Soon, Seoul 06560 (KR); LEE, Hyeong Min, Suwon-si Gyeonggi-do 16705 (KR); JEON, Hyun Ju, Hwaseong-si Gyeonggi-do 18475 (KR); CHOI, Leo Sung Wong, Yongin-si Gyeonggi-do 16918 (KR)
(74) Representative: Cabinet Nony
(86) International application number: PCT/KR2022/008093
(87) International publication number: WO 2022/260435

(57) **Abstract**

The present invention relates to a pharmaceutical composition for preventing or treating degenerative eye diseases, comprising a pyrano[2,3-f]chromen derivative compound, a pharmaceutically acceptable salt thereof, or a solvate thereof.

## Description

### Technical Field

This application claims the benefit of the filing date of Korean Patent Application No. 10-2021-0074192, filed with the Korea Intellectual Property Office on June 8, 2021, the entire content of which is included in the present invention.

The present invention relates to a pharmaceutical composition for preventing or treating degenerative eye disease.

### Background Art

Macular degeneration is a disease that causes blindness by inducing degeneration of the retina, and is a serious age-related ophthalmic disease that can cause blindness. Macular degeneration is classified into wet macular degeneration and dry macular degeneration. Wet macular degeneration (wet AMD, wet age-related macular degeneration) refers to degeneration of the retinal macula associated with neovascularization, which is also called neovascular macular degeneration. Meanwhile, macular degeneration not associated with neovascularization is referred to as dry macular degeneration (dry AMD).

For the treatment of neovascular age-related macular degeneration, a method of injecting a drug for blocking vascular endothelial growth factor (VEGF) into the eye is mainly used. Representatively, ranibizumab (Lucentis^{®}) has been reported to be an effective and safe treatment for patients with neovascular age-related macular degeneration through various clinical studies and is widely used around the world. However, as the lesion frequently recurs, the number of injections increases, placing a significant burden on the patient. In some patients, the lesion does not improve despite injection. Meanwhile, blocking vascular endothelial growth factor (VEGF) interferes with cardiac coronary artery relaxation and blood circulation, and there is a risk of serious side effects in groups at high risk of cardiovascular diseases. Accordingly, the present invention is intended to propose a new therapeutic composition that reduces the risk of side effects and provides an efficient treatment effect on macular degeneration.

### DISCLOSURE

### Technical Problem

The present invention is intended to provide a pharmaceutical composition for preventing or treating degenerative eye disease.

However, objects to be solved by the present invention are not limited to the above-mentioned problems, and other problems not mentioned above will be clearly understood by those skilled in the art from the following description.

### Technical Solution

One embodiment of the present invention provides a pharmaceutical composition for preventing or treating degenerative eye disease containing a pyrano[2,3-f]chromene derivative compound of the following Formula I, a pharmaceutically acceptable salt thereof, or a solvate thereof:

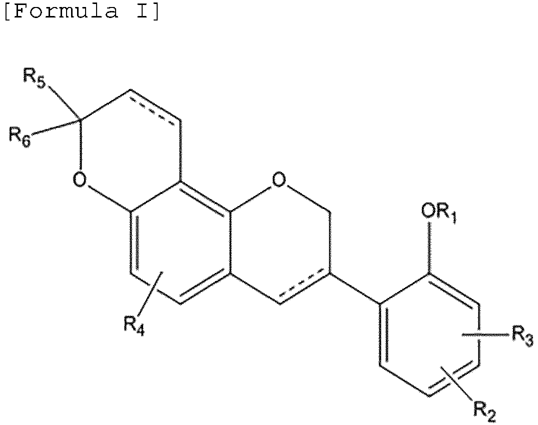

In Formula I above,
the dotted line represents an optional double bond;
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

### Advantageous Effects

The pharmaceutical composition containing a pyrano[2,3-f]chromene derivative compound, a pharmaceutically acceptable salt thereof, or a solvate thereof according to one embodiment of the present invention has an excellent effect of preventing or treating degenerative eye diseases, especially age-related macular degeneration.

Effects of the present invention are not limited to the above-described effects, and effects not mentioned above will be clearly understood by those skilled in the art from the present specification and the accompanying drawings.

### Brief Description of Drawings

FIG. 1 shows the results of obtaining 3D images of lesions in wet-AMD animal model using Z-stack imaging technique and quantifying the volumes of the lesions, according to Experimental Example 1.
FIG. 2 shows the results of measuring mitochondrial reactive oxygen species in wet-AMD animal model according to Experimental Example 2.
FIG. 3 shows the results of obtaining 3D images of lesions in wet-AMD animal model using Z-stack imaging technique and quantifying the volumes of the lesions, according to Experimental Example 3.
FIG. 4 shows the results of measuring mitochondrial reactive oxygen species in wet-AMD animal model according to Experimental Example 4.
FIG. 5 shows the results of obtaining 3D images of lesions in wet-AMD animal model using Z-stack imaging technique and quantifying the volumes of the lesions, according to Experimental Example 5.
FIG. 6 shows the results of measuring mitochondrial reactive oxygen species in wet-AMD animal model according to Experimental Example 4.
FIG. 7 shows the results of obtaining 3D images of lesions in wet-AMD animal model using Z-stack imaging technique and quantifying the volumes of the lesions, according to Experimental Example 7.
FIG. 8 shows the results of measuring mitochondrial reactive oxygen species in wet-AMD animal model according to Experimental Example 8.
FIG. 9 shows the results of measuring eyeball lesions in dry-AMD animal model according to Experimental Example 9.
FIG. 10 shows the results of measuring the cytoskeleton of RPE cells in dry-AMD animal model according to Experimental Example 10.
FIG. 11 shows the results of measuring mitochondrial reactive oxygen species in dry-AMD animal models according to Experimental Example 11.
FIG. 12 shows the results of measuring changes in inflammatory factors in RPE cells in dry-AMD animal model according to Experimental Example 12.

### Best Mode

All technical terms used in the present specification have the same meanings as commonly understood by those skilled in the art to which the present invention pertains, unless otherwise defined. In addition, although preferred methods and samples are described in the present specification, similar or equivalent methods and samples are also included in the scope of the present invention.

Hereinafter, the present invention will be described in more detail.

One embodiment of the present invention provides a pharmaceutical composition for preventing or treating degenerative eye disease containing a pyrano[2,3-f]chromene derivative compound of the following Formula I, a pharmaceutically acceptable salt thereof, or a solvate thereof:

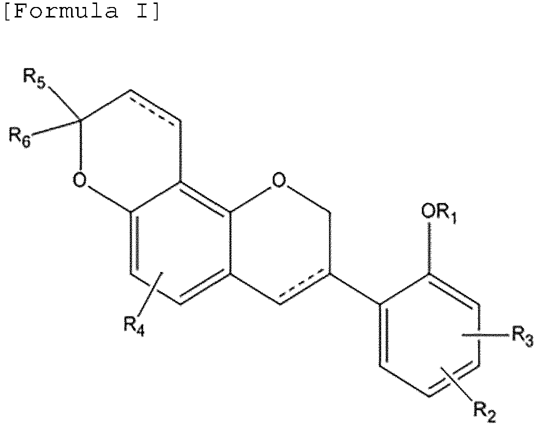

In Formula I above, the dotted line represents an optional double bond; R₁ is any one of a hydrogen atom and a substituted or unsubstituted linear or branched C₁-C₆ alkyl group; R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

The above-mentioned "optional double bond" means that it may be a single bond or a double bond as the case may be.

The pharmaceutical composition containing a pyrano[2,3-f]chromene derivative compound, a pharmaceutically acceptable salt thereof, or a solvate thereof according to one embodiment of the present invention has an excellent effect of preventing or treating degenerative eye diseases, especially age-related macular degeneration.

The pharmaceutical composition containing a pyrano[2,3-f]chromene derivative compound, a pharmaceutically acceptable salt thereof, or a solvate thereof according to one embodiment of the present invention has an excellent effect of preventing or treating degenerative eye diseases that have a significant impact on vision, such as cataracts, glaucoma, diabetic retinopathy, and macular degeneration. Among the degenerative eye diseases, macular degeneration may be wet macular degeneration or dry macular degeneration.

According to one embodiment of the present invention, R₁ may be a hydrogen atom, a methyl group, or an ethyl group, R₂ may be a methyl group, an ethyl group, a n-propyl group, an isopropyl group, a n-butyl group, an iso-butyl group, a tert-butyl group, a methoxy group, an ethoxy group, a n-propoxy group, an iso-propoxy group, a n-butoxy group, an iso-butoxy group, a tert-butoxy group, or a hydroxyl group, R₃ and R₄ may be each independently a hydrogen atom, and R₅ and R₆ may be each independently a methyl group. When R₁ and R₆ are those described above, the pharmaceutical composition containing a pyrano[2,3-f]chromene derivative compound of Formula I, a pharmaceutically acceptable salt thereof, or a solvate thereof may be chemically stable and have an excellent effect of preventing or treating degenerative eye disease.

According to one embodiment of the present invention, the pyrano[2,3-f]chromene derivative compound of Formula I may comprise a compound of the following Formula I-1:

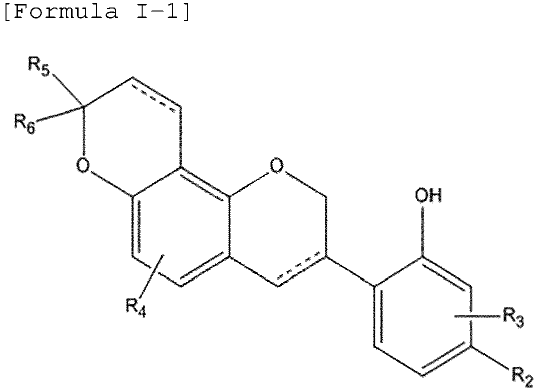

In Formula I-1 above, the dotted line represents an optional double bond; R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula I-1.

The compound of Formula I-1 is a compound wherein R₁ in Formula I is a hydrogen atom and R₂ is fixed at a specific position. A pharmaceutical composition containing the pyrano[2,3-f]chromene derivative compound represented by Formula I-1, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise a compound of the following Formula II:

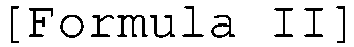

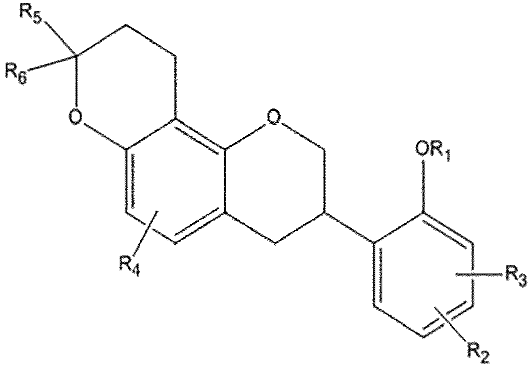

In Formula II above, R₁ is any one of a hydrogen atom and a substituted or unsubstituted linear or branched C₁-C₆ alkyl group; R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula II.

A pharmaceutical composition containing the pyrano[2,3-f]chromene derivative compound represented by Formula II among Formula I, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise a compound of the following Formula II-1:

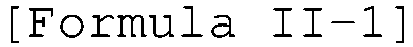

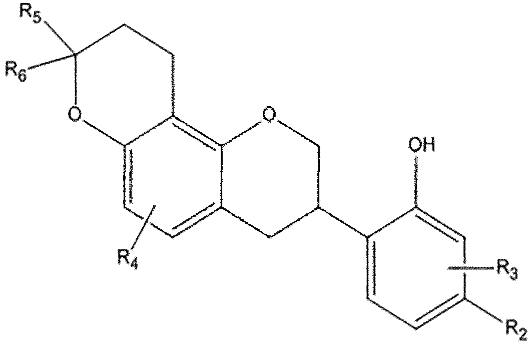

In Formula II-1 above, R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula II.

The compound of Formula II-1 is a compound wherein R₁ in Formula II is a hydrogen atom and R₂ is fixed at a specific position. A pharmaceutical composition containing the pyrano[2,3-f]chromene derivative compound represented by Formula II-1, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise a compound of the following Formula II-1a:

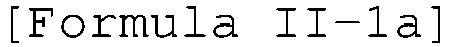

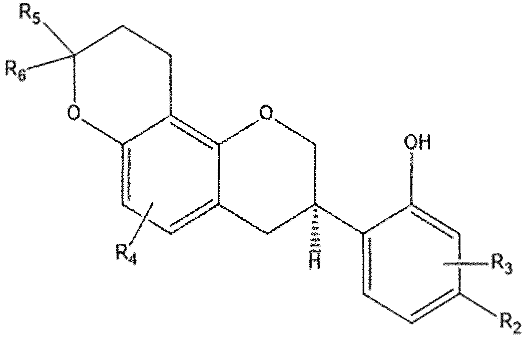

In Formula II-1a above, R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula II-1a.

The compound of Formula II-1a corresponds to the (S) isomer of the compound of Formula II-1. As the compound represented by Formula II-1a has optical activity, a pharmaceutical composition containing the pyrano[2,3-f]chromene derivative compound represented by Formula II-1a, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise a compound of the following Formula III:

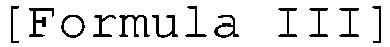

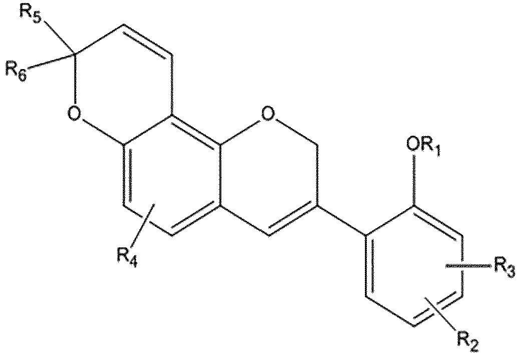

In Formula III above, R₁ is any one of a hydrogen atom and a substituted or unsubstituted linear or branched C₁-C₆ alkyl group; R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula III.

A pharmaceutical composition containing the pyrano[2,3-f]chromene derivative compound represented by Formula III, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise a compound of the following Formula III-1:

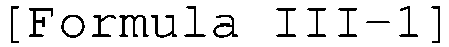

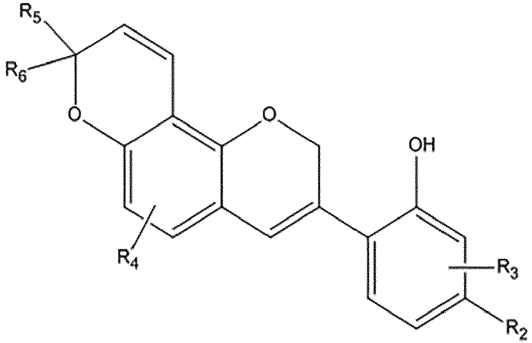

In Formula III-1 above, R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group; R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom; R₄ is a hydrogen atom, methyl, ethyl, methoxy, or ethoxy; and R₅ and R₆ are each independently any one of a hydrogen atom and a C₁-C₆ alkyl group, wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group. Specifically, the pyrano[2,3-f]chromene derivative compound of Formula I may be the compound of Formula III-1.

The compound of Formula III-1 is a compound wherein R₁ in Formula III is a hydrogen atom and R₂ is fixed at a specific position. A pharmaceutical composition containing the pyrano[2,3-f]chromene derivative compound represented by Formula III-1, a pharmaceutically acceptable salt thereof, or a solvate thereof may be chemically stable and have an excellent effect of preventing or treating degenerative eye disease. In particular, it may have an excellent effect of preventing and treating age-related macular degeneration.

According to one embodiment of the present invention, the pyrano[2,3-f]chromene derivative compound of Formula I may comprise at least one of the following Compounds 1-1 to 1-18:

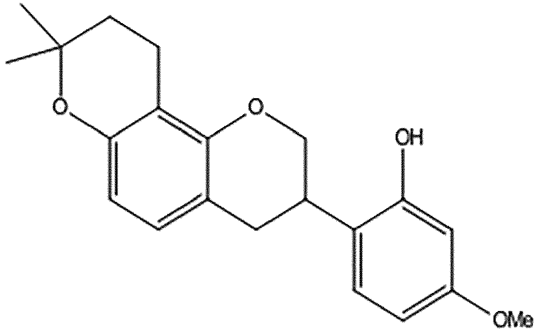

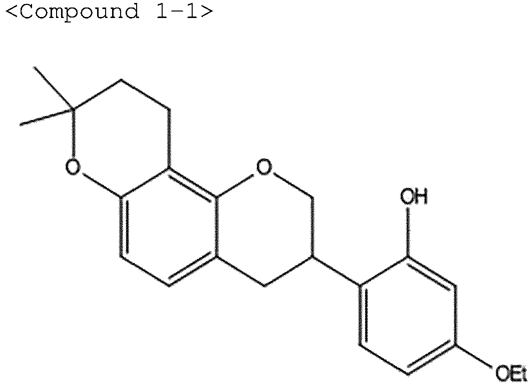

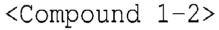

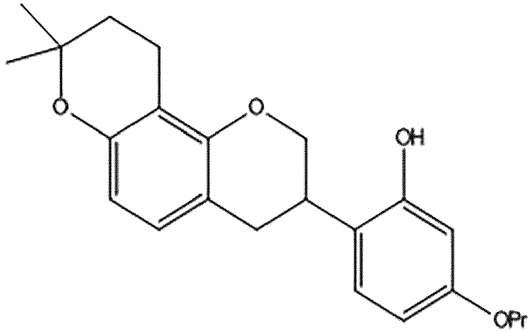

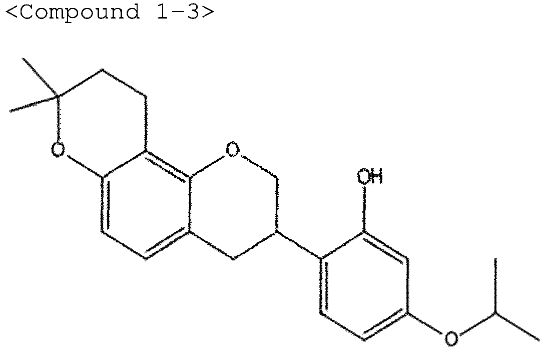

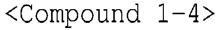

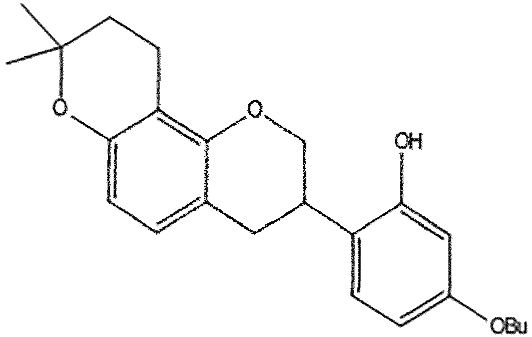

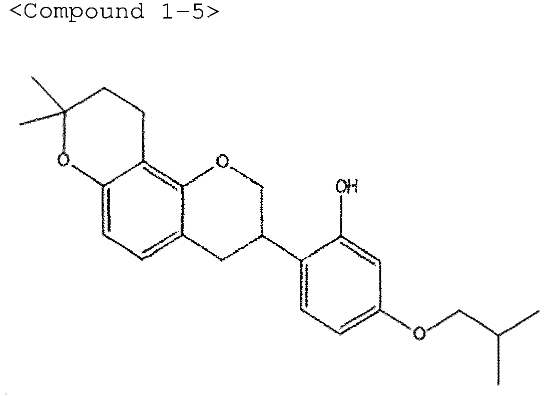

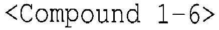

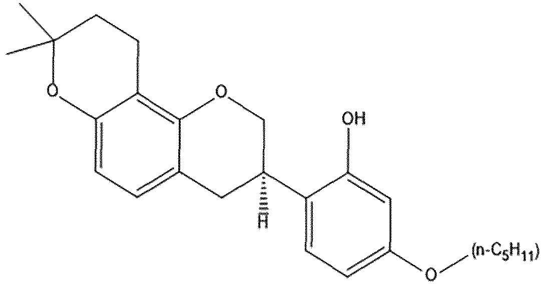

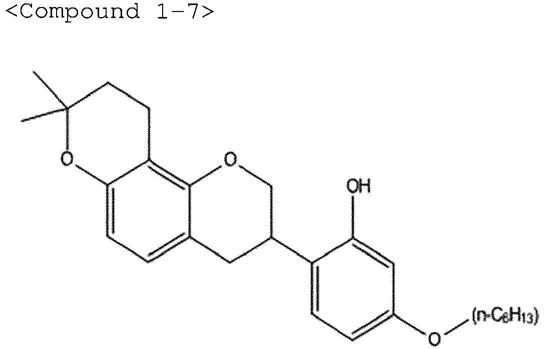

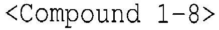

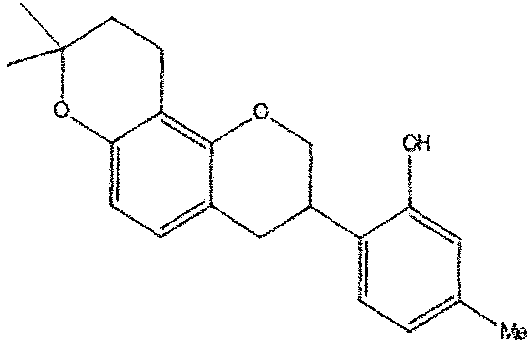

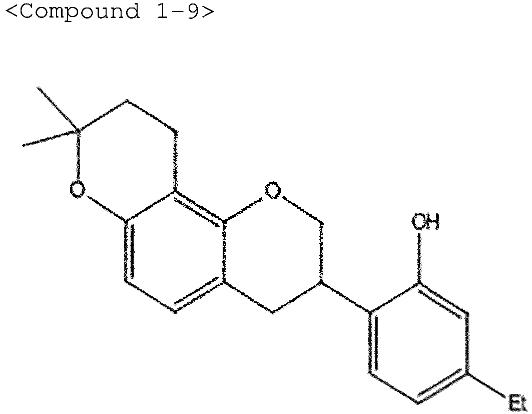

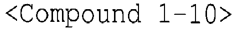

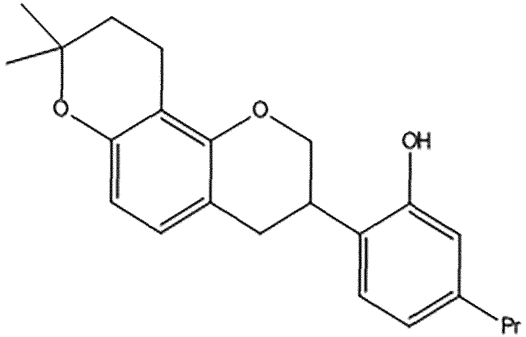

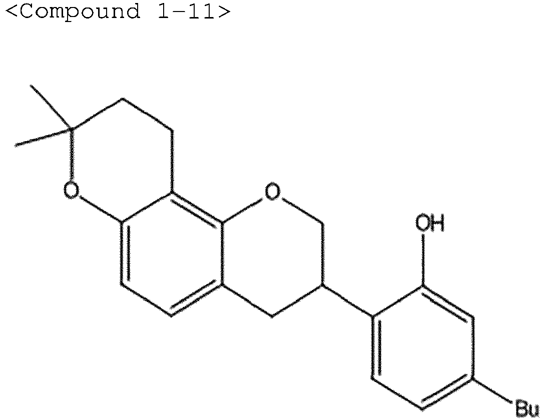

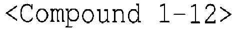

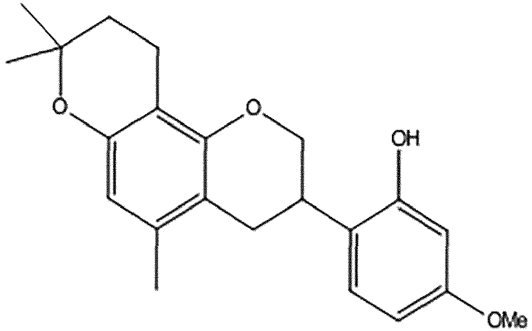

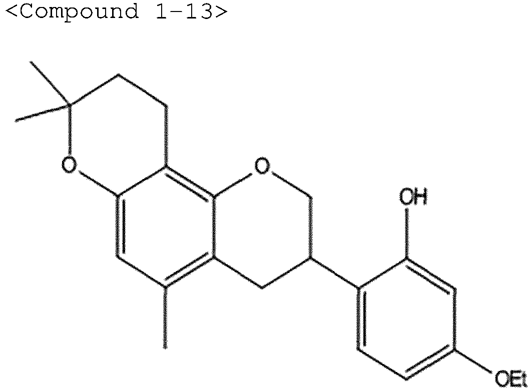

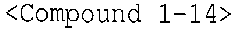

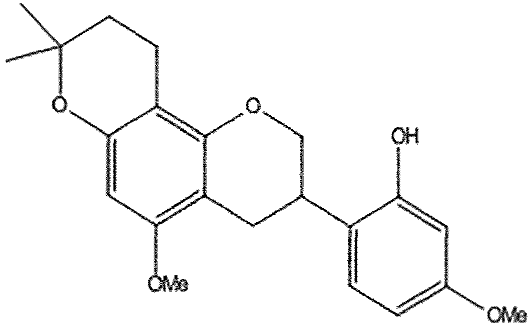

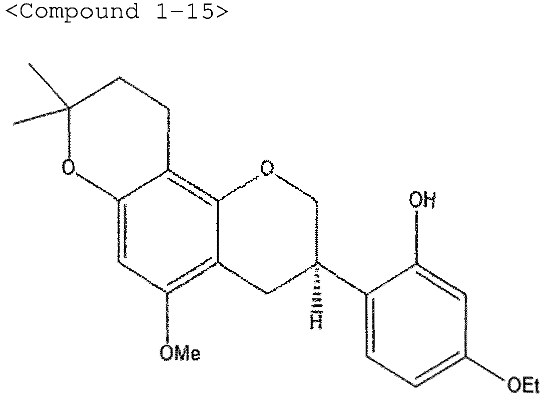

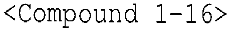

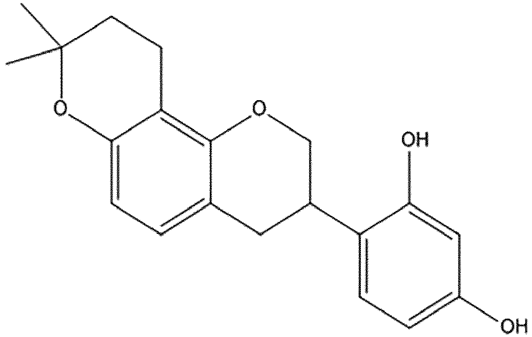

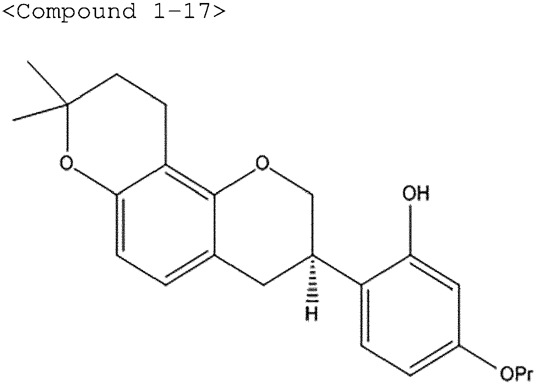

### <Compound 1-18>

Specifically, among the above compounds, Compound 1-2 and Compound 1-18 may have excellent effects of preventing and treating degenerative eye diseases, especially age-related macular degeneration.

According to one embodiment of the present invention, the pyrano[2,3-f]chromene derivative compound of Formula I may comprise at least one of the following Compounds 1-1a to 1-17a:

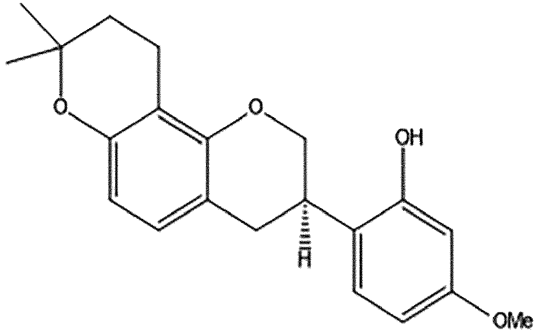

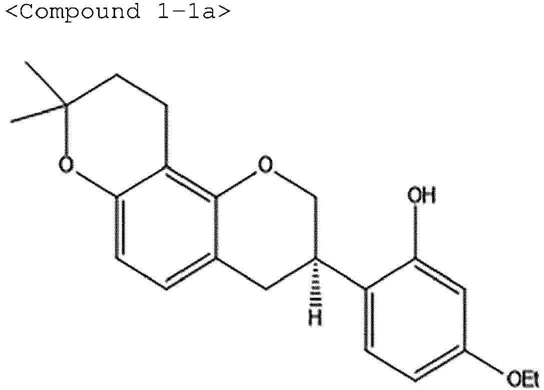

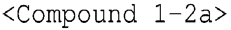

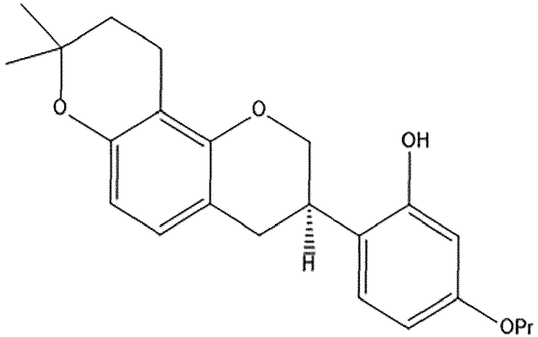

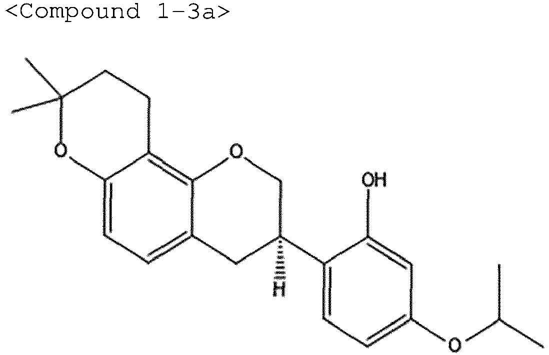

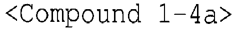

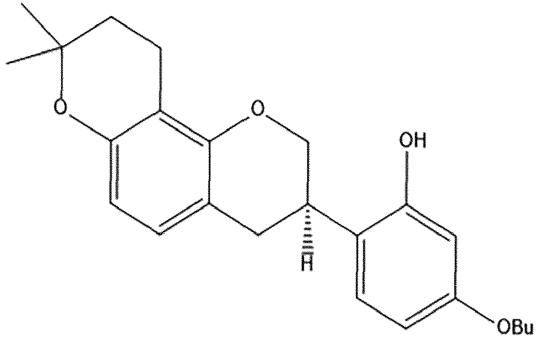

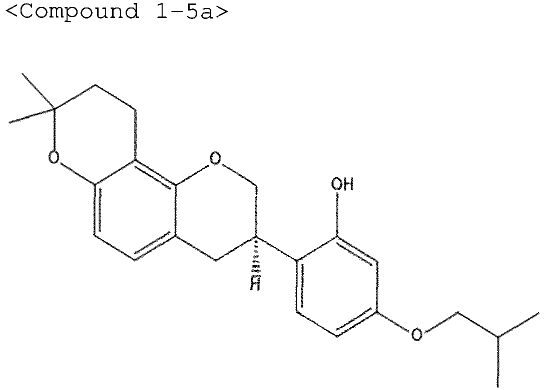

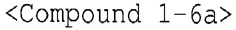

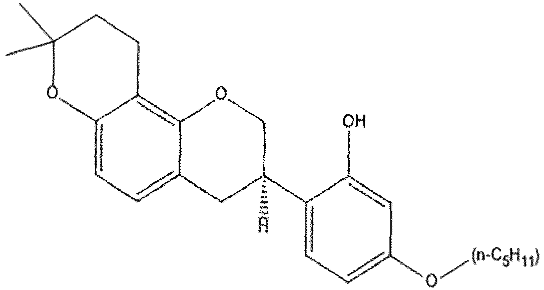

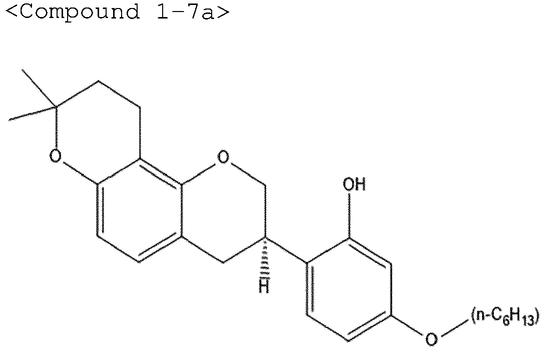

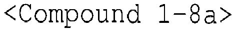

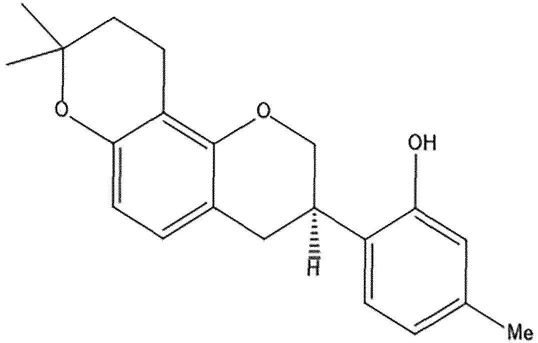

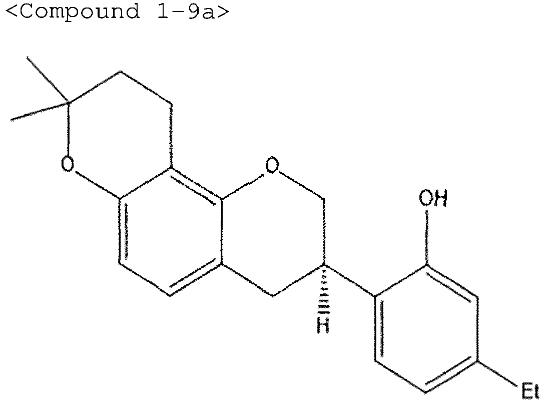

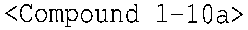

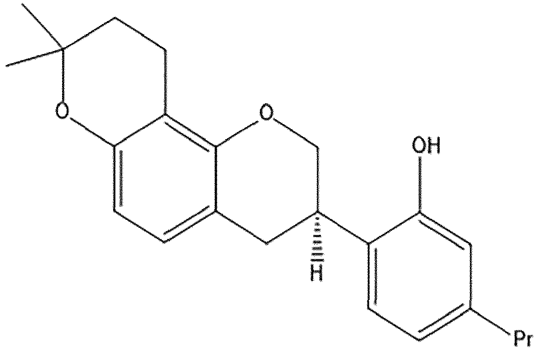

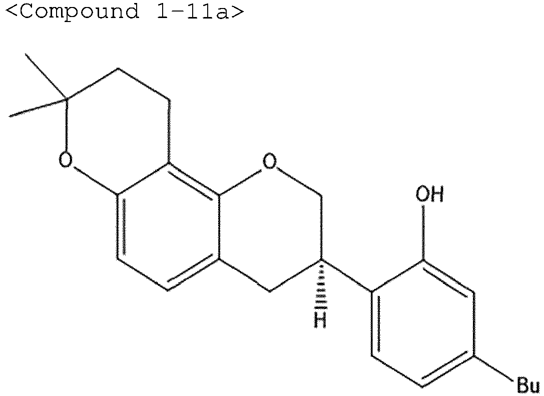

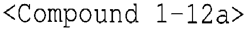

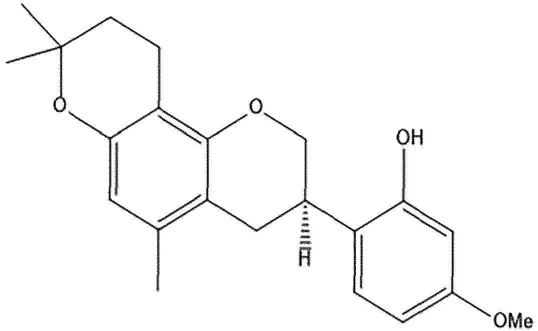

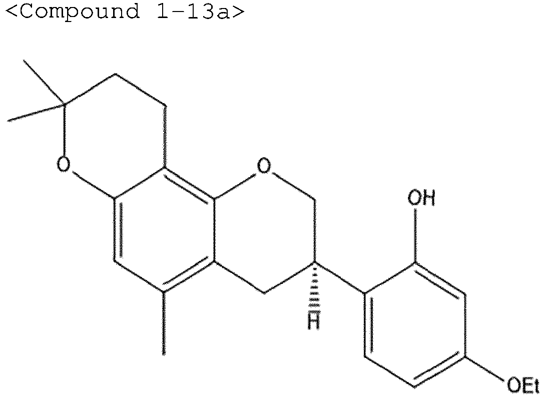

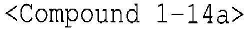

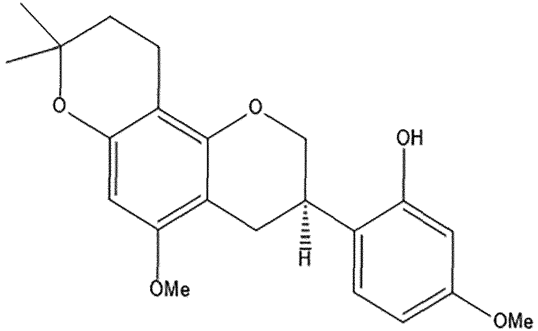

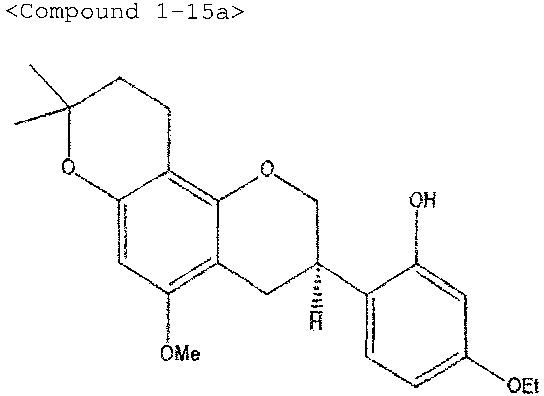

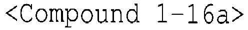

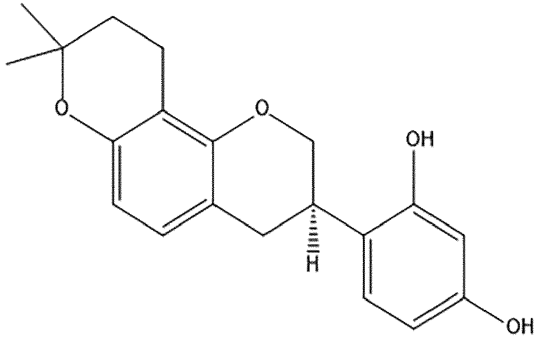

### <Compound 1-17a>

Here, Compound 1-3a is the same as Compound 1-18.

According to one embodiment of the present invention, the pyrano[2,3-f]chromene derivative compound of Formula I may comprise at least one of the following Compounds 2-1 to 2-17:

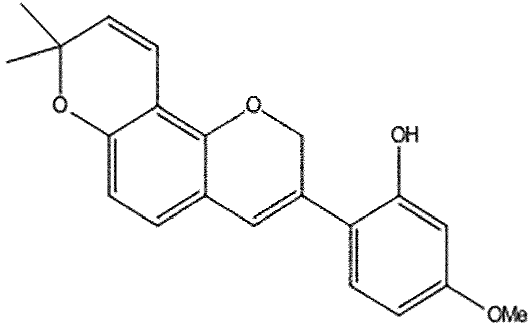

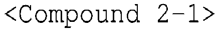

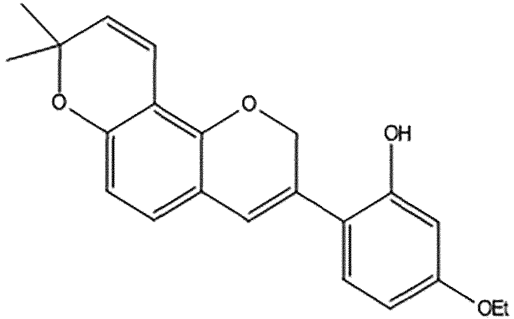

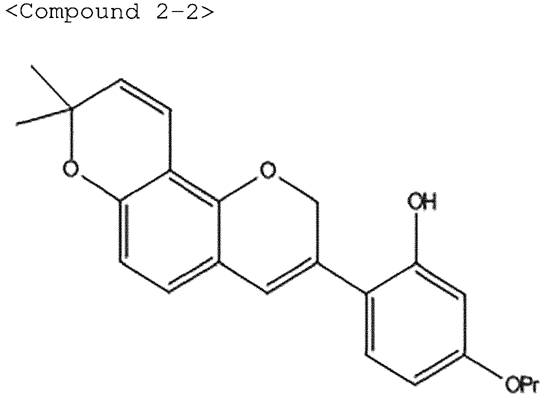

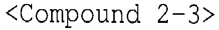

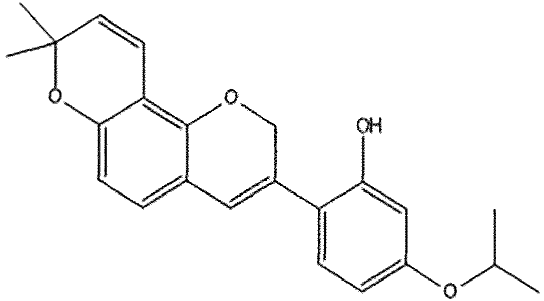

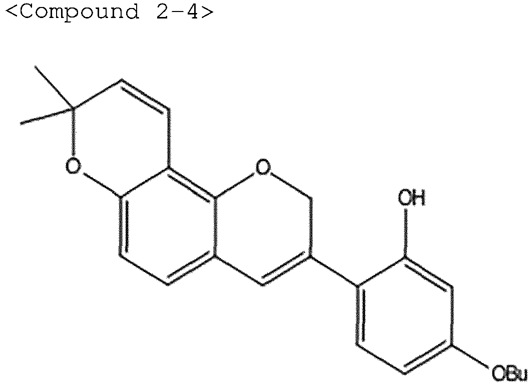

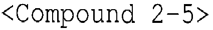

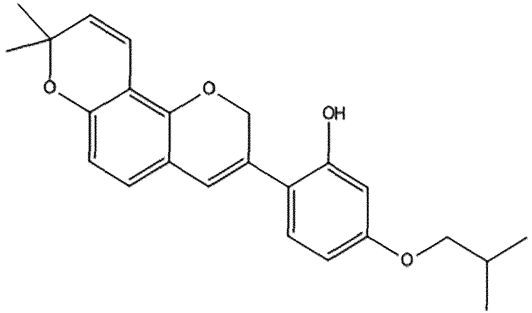

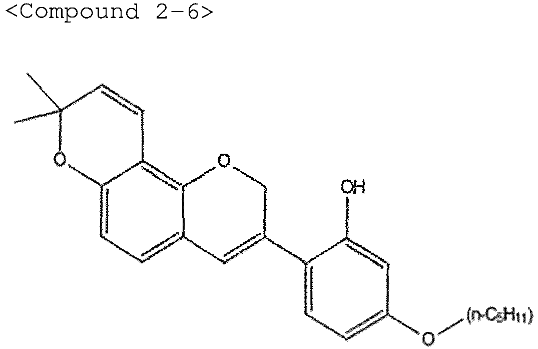

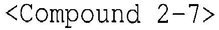

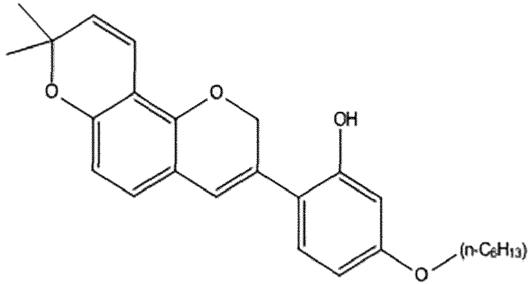

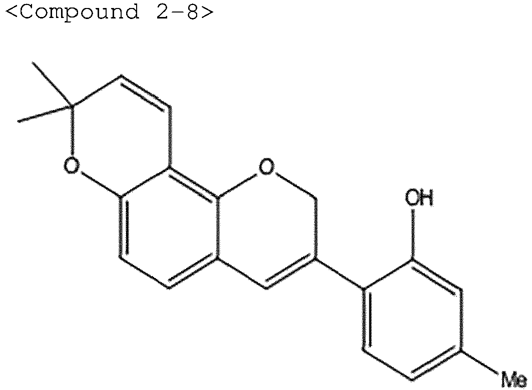

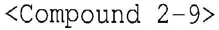

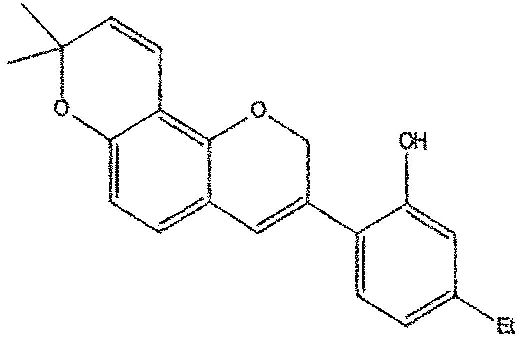

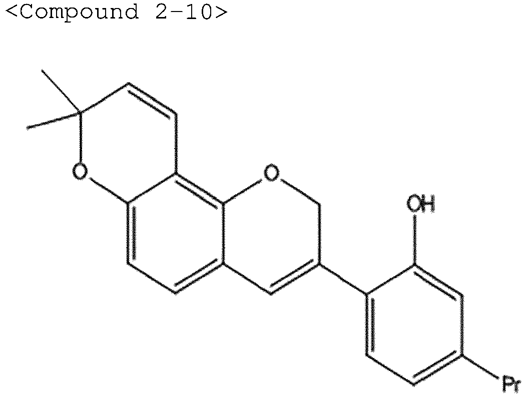

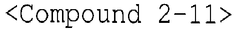

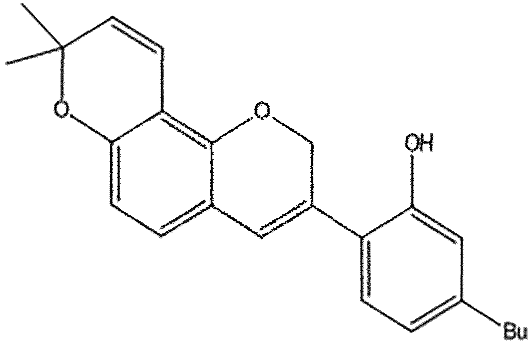

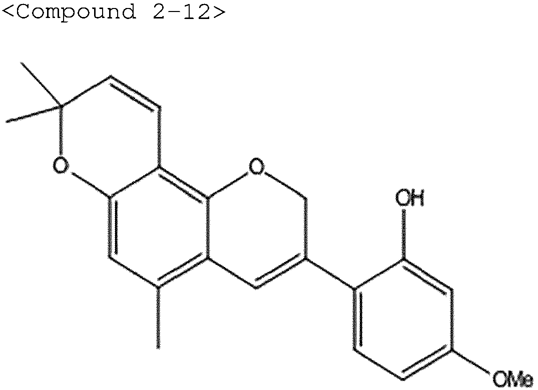

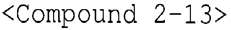

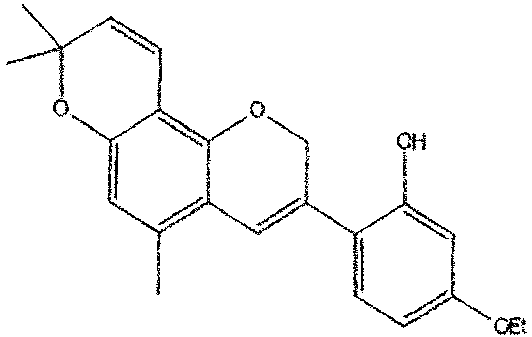

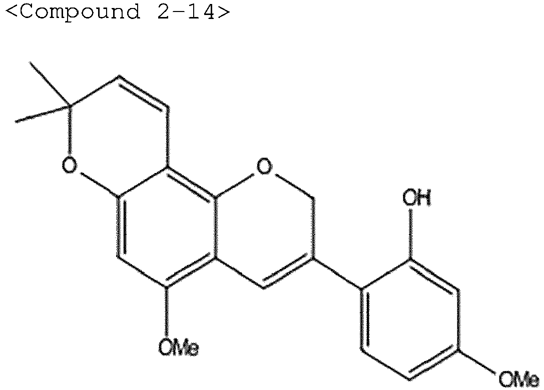

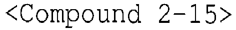

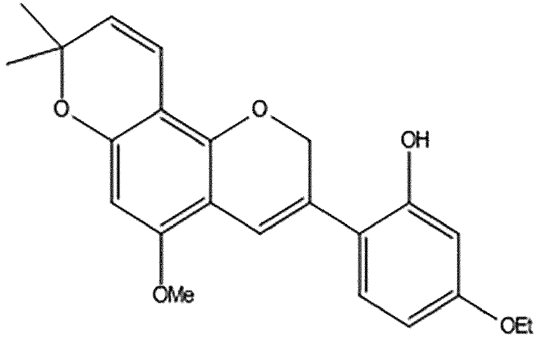

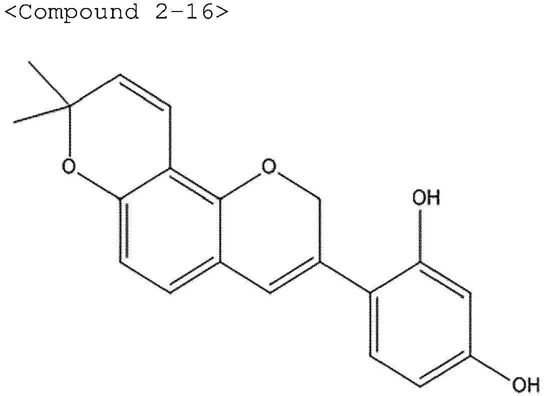

### <Compound 2-17>

One embodiment of the present invention provides a method for preventing or treating degenerative eye disease comprising a step of administering, to a subject suspected of having degenerative eye disease, a composition containing a pyrano[2,3-f]chromene derivative compound of the following Formula I, a pharmaceutically acceptable salt thereof, or a solvate thereof:

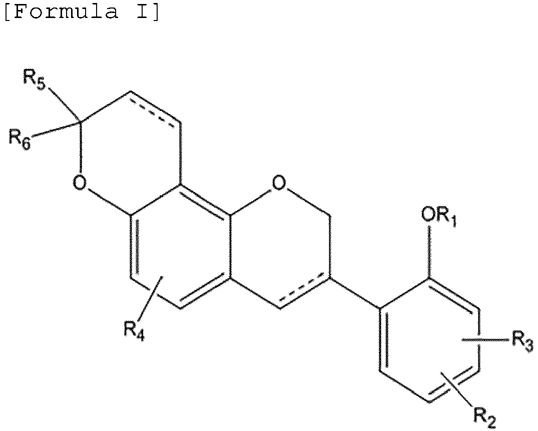

In Formula I above,
the dotted line represents an optional double bond;
R₁ is any one of a hydrogen atom and a substituted or unsubstituted linear or branched C₁-C₆ alkyl group;
**R₂** is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

One embodiment of the present invention provides the use of a composition containing a pyrano[2,3-f]chromene derivative compound of the following Formula I, a pharmaceutically acceptable salt thereof, or a solvate thereof for preventing or treating degenerative eye disease:

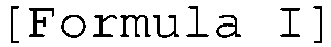

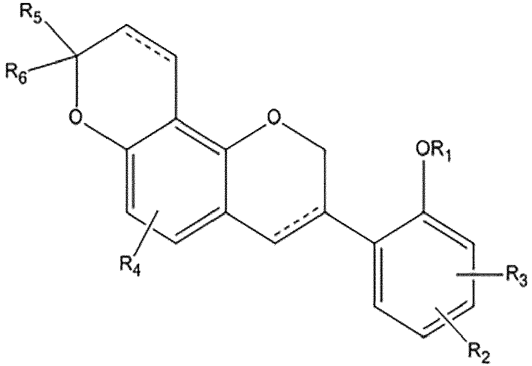

In Formula I above,
the dotted line represents an optional double bond;
R₁ is any one of a hydrogen atom and a substituted or unsubstituted linear or branched C₁-C₆ alkyl group;
**R₂** is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

According to one embodiment of the present invention, the degenerative eye disease may be age-related macular degeneration. The age-related macular degeneration may be either wet macular degeneration (wet AMD, wet age-related macular degeneration), which is degeneration of the retinal macula associated with neovascularization, or dry macular degeneration (dry AMD) which is macular degeneration not associated with neovascularization. In particular, as described above, the pharmaceutical composition containing the pyrano[2,3-f]chromene derivative compound of Formula I, a pharmaceutically acceptable salt thereof, or a solvate thereof may have an excellent effect of preventing and treating age-related macular degeneration.

According to one embodiment of the present invention, the pyranopyrano[2,3-f]chromene derivative compound of Formula I can protect mitochondrial function from damage by effectively removing mitochondrial ROS from RPE cells, and prevent the detachment of RPE cells by restoring the structure of RPE cells. In addition, it can induce anti-inflammatory responses by suppressing the production of pro-inflammatory cytokines, suggesting that it may be used to prevent or treat degenerative eye diseases, especially age-related macular degeneration.

According to one embodiment of the present invention, the pharmaceutically acceptable salt of the compound of Formula I may mean that the compound of Formula I forms a salt with a free acid and exists as an acid addition salt. A pharmaceutically acceptable acid addition salt of the compound of Formula 1 may be formed according to a conventional method known in the art. An organic acid or an inorganic acid may be used as the free acid. As the inorganic acid, hydrochloric acid, hydrobromic acid, sulfuric acid, phosphoric acid or the like may be used, and as the organic acid, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, galacturonic acid, embonic acid, glutamic acid, aspartic acid, or the like may be used.

The pharmaceutical composition according to one embodiment of the present invention may contain, in addition to the pyrano[2,3-f]chromene derivative compound of Formula I, a pharmaceutically acceptable salt thereof, or a solvate thereof, pharmaceutically acceptable carriers, excipients or diluents. The pharmaceutically acceptable carriers, excipients or diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

In one embodiment of the present invention, the pyrano[2,3-f]chromene derivative compound of Formula I may be produced by the same method as described in Korean Patent Application Publication Nos. 10-2015-0075030 and 10-2018-0002539, without being limited thereto.

According to one embodiment of the present invention, the pharmaceutical composition may be formulated in conventional pharmaceutical dosage forms known in the art. The pharmaceutical composition may be formulated and administered in any dosage forms including, but not limited to, oral dosage forms, injections, suppositories, transdermal dosage forms, and intranasal dosage forms. Preferably, it may be formulated in an oral dosage form or as an injection.

When the pharmaceutical composition is formulated in each of the above-described dosage forms, the formulation may be prepared by adding a pharmaceutically acceptable carrier necessary for the preparation of each dosage form. As used herein, the term "pharmaceutically acceptable carrier" is used to refer to any component other than a pharmaceutically active ingredient. The term "pharmaceutically acceptable" refers to properties that do not cause any pharmaceutically undesirable change resulting from interaction with other components present in the compositions (for example, interaction between carriers or interaction between a pharmaceutically active ingredient and a carrier). The choice of the pharmaceutically acceptable carrier may depend on factors such as the properties of a particular dosage form, the mode of administration, and the effects of the carrier on solubility and stability.

According to one embodiment of the present invention, the pharmaceutically acceptable carrier contained in the pharmaceutical composition for oral administration may be at least one selected from the group consisting of a diluent, a binder, a disintegrant, a glidant (or lubricant), an adsorbent, a stabilizer, a solubilizing agent, a sweetener, a colorant, and a flavoring agent, without being limited thereto.

The term "diluent" refers to any excipient that is added in order to make a dosage form having a suitable size by increasing the volume of the composition. The diluent may be at least one selected from among starch (e.g., potato starch, corn starch, wheat starch, pre-gelatinized starch), microcrystalline cellulose (e.g., low-hydration microcrystalline cellulose), lactose (e.g., lactose monohydrate, anhydrous lactose, spraydried lactose), glucose, sorbitol, mannitol, sucrose, alginate, alkaline earth metal salts, clay, polyethylene glycol, dicalcium phosphate, anhydrous calcium hydrogen phosphate, and silicon dioxide, without being limited thereto. In the present invention, the diluent may be used in an amount of 5 wt% to 50 wt% based on the total weight of the pharmaceutical composition. For example, the diluent may be used in an amount of 10 wt% to 35 wt% based on the total weight of the pharmaceutical composition in order to ensure tabletting and maintain quality.

The term "binder" refers to a material that is used to impart stickiness to a powdery material, making compression easy and improving fluidity. The binder may be at least one selected from among starch, microcrystalline cellulose, highly dispersible silica, mannitol, lactose, polyethylene glycol, polyvinylpyrrolidone, cellulose derivatives (e.g., hydroxypropylmethyl cellulose, hydroxypropyl cellulose, low-substituted hydroxypropyl cellulose), natural gum, synthetic gum, povidone, copovidone, and gelatin, without being limited thereto. In the present invention, the binder may be used in an amount of 2 wt% to 15 wt% based on the total weight of the pharmaceutical composition. For example, the binder may be used in an amount of 1 wt% to 3 wt% based on the total weight of the pharmaceutical composition in order to ensure tabletting and maintain quality.

The term "disintegrant" refers to a material that is added to facilitate the breakdown or disintegration of a solid dosage form after administration *in vivo.* Examples of the disintegrant include, but are not limited to, starch, such as sodium starch glycolate, corn starch, potato starch, or pre-gelatinized starch, or modified starch; clay, such as bentonite, montmorillonite or veegum; cellulose, such as microcrystalline cellulose, hydroxypropyl cellulose or carboxymethyl cellulose; algins, such as sodium alginate or alginic acid; crosslinked cellulose, such as croscarmellose sodium; gum, such as guar gum or xanthan gum; a crosslinked polymer such as crosslinked polyvinylpyrrolidone (crospovidone); or an effervescent agent such as sodium bicarbonate or citric acid, which may be used alone or in combination. In the present invention, the disintegrant may be used in an amount of 2 wt% to 15 wt% based on the total weight of the pharmaceutical composition. For example, the disintegrant may be used in an amount of 4 wt% to about 10 wt% based on the total weight of the pharmaceutical composition in order to ensure tabletting and maintain quality.

The term "glidant or lubricant" refers to a material that functions to prevent the adhesion of powder to a compression system and improve the fluidity of granules. Examples of the glidant include, but are not limited to, hard anhydrous silicic acid, talc, stearic acid, a metal salt (magnesium salt or calcium salt) of stearic acid, sodium lauryl sulfate, hydrogenated vegetable oil, sodium benzoate, sodium stearyl fumarate, glyceryl behenate, glyceryl monostearate, and polyethylene glycol, which may be used alone or in combination. In the present invention, the glidant may be used in an amount of 0.1 wt% to 5 wt% based on the total weight of the pharmaceutical composition. For example, the glidant may be used in an amount of 1 wt% to 3 wt% in order to ensure tabletting and maintain quality.

Examples of the adsorbent include, but are not limited to, hydrated silicon dioxide, hard anhydrous silicic acid, colloidal silicon dioxide, magnesium aluminometasilicate, microcrystalline cellulose, lactose, and crosslinked polyvinylpyrrolidone, which may be used alone or in combination.

The stabilizer may be at least one selected from among antioxidants such as butylhydroxyanisole, butylhydroxytoluene, carotene, retinol, ascorbic acid, tocopherol, tocopherolpolyethylene glycol succinic acid or propyl gallate; cyclic sugar compounds such as cyclodextrin, carboxyethyl cyclodextrin, hydroxypropyl cyclodextrin, sulfobutyl ether or cyclodextrin; and organic acids such as phosphoric acid, lactic acid, acetic acid, citric acid, tartaric acid, succinic acid, maleic acid, fumaric acid, glycolic acid, propionic acid, gluconic acid or glucuronic acid, without being limited thereto

Optionally, the pharmaceutical composition may contain known additives that enhance palatability by stimulating taste. For example, a sweetener such as sucralose, sucrose, fructose, erythritol, potassium acesulfame, sugar alcohol, honey, sorbitol or aspartame may be added to more effectively mask bitterness and maintain the stability and quality of the formulation of the composition. In addition, the composition may contain an acidifier agent such as citric acid or sodium citrate; a natural flavoring agent such as a plum flavor, a lemon flavor, a pineapple flavor or a herb flavor; or a natural pigment such as natural fruit juice, chlorophyllin or a flavonoid.

The pharmaceutical composition for oral administration may be in the form of a solid, semi-solid or liquid formulation for oral administration. Examples of the solid formulation for oral administration include, but are not limited to, tablets, pills, hard or soft capsules, powders, fine granules, granules, powders for reconstitution of a solution or suspension, lozenges, wafers, oral strips, dragees, and chewing gum. Examples of the liquid formulation for oral administration include solutions, suspensions, emulsions, syrups, elixirs, alcoholic solutions, aromatic water, lemonade preparations, extracts, infusodecoctions, tinctures, and medicated spirits. Examples of the semi-solid formulation include, but are not limited to, aerosols, creams, gels, and the like.

The pharmaceutical composition according to the present invention may be formulated as an injection, and when it is formulated as an injection, it may contain, as a diluent, a nontoxic buffer solution isotonic with blood, for example, a phosphate buffered saline of pH 7.4. The pharmaceutical composition may contain other diluents or additives, in addition to the buffered saline.

The carrier used in the above-mentioned formulation and a method for preparation of the formulation may be selected and prepared according to a method well known in the art, and for example, may be prepared according to the method described in Remington's Pharmaceutical Science (latest edition).

The dosage and timing of administration of the pharmaceutical composition according to the present invention may vary depending on the subject's age, sex, condition and body weight, the route of administration, the frequency of administration, and the type of drug. The daily dosage of the active ingredient, that is, the compound of Formula I, is about 0.1 to 300 mg/kg, preferably 1 to 200 mg/kg. The dosage may be appropriately increased or decreased depending on the kind of disease, the degree of progression of the disease, the route of administration, the subject's sex, age and body weight, and the like
The pharmaceutical composition according to the present invention may be arbitrarily administered several times so that the total daily dosage of the compound of Formula I as an active ingredient is 1 to 20 mg/kg for an adult, in order to obtain the desired effect.

The pharmaceutical composition according to the present invention may contain the compound of Formula I according to the present invention in an amount of about 1 to 50 wt%, preferably 10 to 40 wt%, based on the total weight of the composition.

### Mode for Invention

Hereinafter, the present invention will be described in detail with reference to examples. However, the examples according to the present invention may be modified in various different forms, and the scope of the present invention is not to be construed as being limited to the examples described below. The examples of the present specification are provided to more completely explain the present disclosure to those of ordinary skill in the art.

### <Experimental Examples>

In order to examine whether the pyrano[2,3-f]chromene derivative compound of Formula 1 is effective in preventing or treating degenerative eye disease, the following experiments were conducted.

The following experiments were conducted on Compound 1-2 and Compound 1-18 produced using the methods described in Korean Patent Application Publication Nos. 10-2015-0075030 and 10-2018-0002539.

### Experimental Example 1: Evaluation of Preventive and Therapeutic Effects in Wet-AMD Model with CNV

Male C57BL/6 mice were anesthetized with zoletil and rompun, and a retinal lesion (wet-AMD) was induced by performing three laser photocoagulations at the 2 o'clock, 6 o'clock, and 10 o'clock positions at on the retina of the mouse eye under a slit-lamp microscope. Each of Compound 1-2 and Compound 1-18 was administered orally starting from 6 days before inducing wet-AMD, and was administered orally for 7 days after inducing the disease. Eylea (Bayer, Germany), used as a positive control, was administered by direct injection into the eye immediately after induction of the disease model.

After weighing each mouse, FITC-dextran (SIGMA, #46944) diluted in saline at a concentration of 5 mg/kg was injected into the tail veins of the mice to which each of Compound 1-2 and Compound 1-18 has been administered for 7 days after inducing the wet-AMD model. 3D images of the lesions were obtained using the Z-stack imaging technique, and the volumes of the lesions on the images were quantitatively measured.

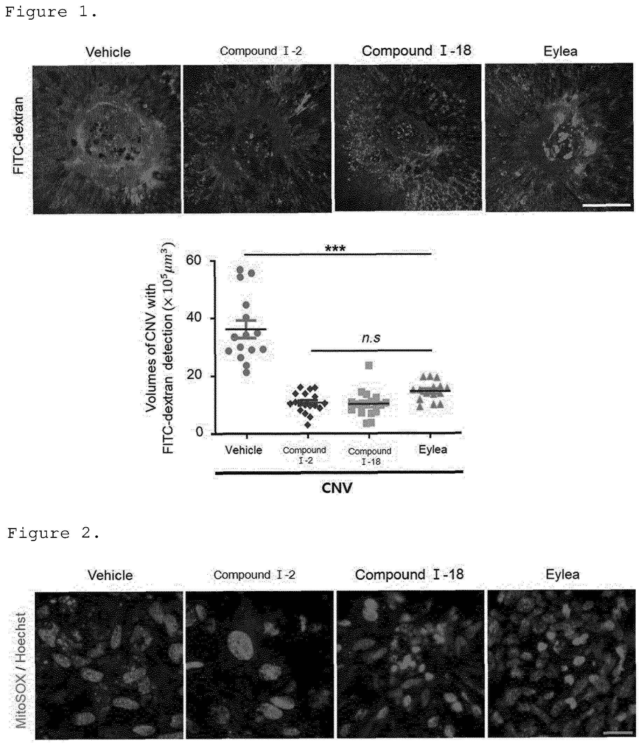

FIG. 1 shows images of the ocular lesions in the wet-AMD animal model and the results of quantitatively measuring the volumes of the lesions, according to Experimental Example 1. Referring to FIG. 1, it can be found through the 3D images and the quantitative reduction in the lesion volume that the administration of each of Compound 1-2 and Compound 1-18 inhibited lesion formation in the wet-AMD model, and exhibited an efficacy equivalent to that of the positive control anti-VEGF agent.

### Experimental Example 2: Analysis of Changes in Mitochondrial Reactive Oxygen Species by Preventive Effect in CNV Animal Model (Wet-AMD)

**RPE** cells were isolated from the same model as in Experimental Example 1 and stained with MitoSOX, and changes in mitochondrial reactive oxygen species in the RPE cells were examined through fluorescence microscopy. Specifically, immediately after enucleation of the mouse eye, mitochondria and nuclei were stained using the MitoSOX^{™} (Invitrogen, #M36008) kit and imaged with a fluorescence microscope at a fluorescence wavelength of 510/580 nm, and changes in reactive oxygen species in the mitochondria were measured.

FIG. 2 shows the results of analyzing changes in mitochondrial reactive oxygen species in the RPE cells from the wet-AMD animal model according to Experimental Example 2. Referring to FIG. 2, it can be found that the administration of each of Compound 1-2 and Compound 1-18 reduced mitochondrial reactive oxygen species in the wet-AMD model, and exhibited an efficacy equivalent to that of the positive control anti-VEGF agent.

### Experimental Example 3: Evaluation of Therapeutic Effect in Wet-AMD Model with CNV

Changes after administering Compound 1-2 for 7 days after induction of the disease without administration before induction of the disease to the wet-AMD mouse model induced in the same manner as in Experimental Example 1 were analyzed. In the same manner as Experimental Example 1, the image of the lesion was obtained and the volume of the lesion was quantified.

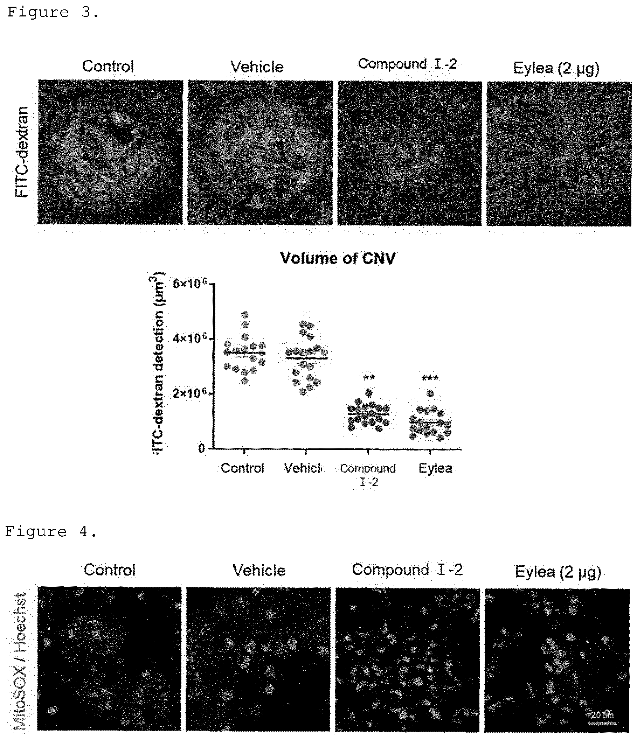

FIG. 3 shows images of the ocular lesions in the wet-AMD animal model and the results of quantitatively measuring the volumes of the lesions, according to Experimental Example 3. Referring to FIG. 3, it can be found through the 3D images and the quantitative reduction in the lesion volume that Compound 1-2 suppressed formation of the lesion even when it was administered after induction of the disease, and exhibited an efficacy equivalent to that of the positive control anti-VEGF agent.

### Experimental Example 4: Analysis of Changes in Mitochondrial Reactive Oxygen Species by Therapeutic Effect in CNV Animal Model (Wet-AMD)

**RPE** cells were isolated from the same model as in Experimental Example 3 and stained with MitoSOX, and changes in mitochondrial reactive oxygen species in the RPE cells were examined through fluorescence microscopy. Measurement was performed in the same manner as in Experimental Example 2.

FIG. 4 shows the results of analyzing changes in mitochondrial reactive oxygen species in the RPE cells from the wet-AMD animal model according to Experimental Example 4. Referring to FIG. 4, it can be found that Compound 1-2 reduced mitochondrial reactive oxygen species in the wet-AMD model even when it was administered after induction of the disease, and exhibited an efficacy equivalent to that of the positive control anti-VEGF agent.

### Experimental Example 5: Evaluation of Effect of Co-Administration with Anti-VEGF Agent in Wet-AMD Model with CNV

Changes after co-administering Compound 1-2 and the anti-VEGF agent (Eylea) for 7 days after induction of the disease without administration before induction of the disease to the wet-AMD mouse model induced in the same manner as in Experimental Example 1 were analyzed. In the same manner as Experimental Example 1, the image of the lesion was obtained and the volume of the lesion was quantified.

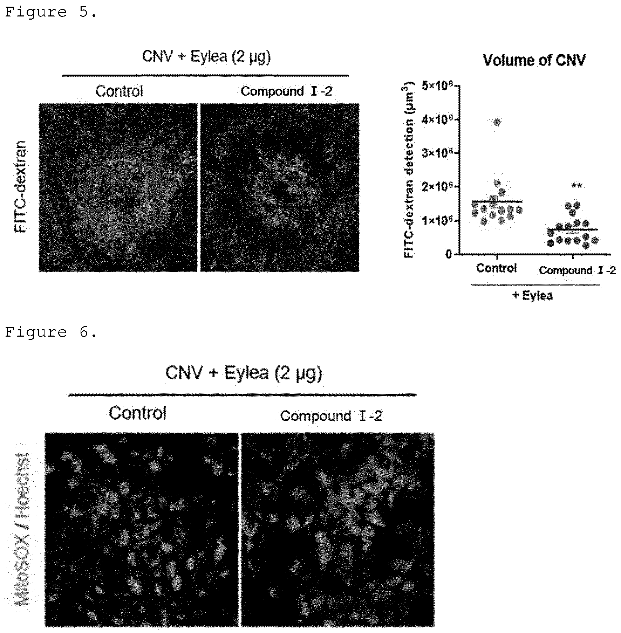

FIG. 5 shows images of the ocular lesions in the wet-AMD animal model and the results of quantitatively measuring the volumes of the lesions, according to Experimental Example 5. Referring to FIG. 5, it can be found through the 3D images and the quantitative reduction in the lesion volume that co-administration of Compound 1-2 and the anti-VEGF agent suppressed formation of the lesion.

### Experimental Example 6: Analysis of Changes in Mitochondrial Reactive Oxygen Species by Co-Administration with Anti-VEGF Agent in CNV Animal Model (Wet-AMD)

RPE cells were isolated from the same model as in Experimental Example 5 and stained with MitoSOX, and changes in mitochondrial reactive oxygen species in the RPE cells were examined through fluorescence microscopy. Measurement was performed in the same manner as in Experimental Example 2.

FIG. 6 shows the results of analyzing changes in mitochondrial reactive oxygen species in the RPE cells from the wet-AMD animal model according to Experimental Example 6. Referring to FIG. 6, it can be found that co-administration of Compound 1-2 and the anti-VEGF agent removed most of mitochondrial reactive oxygen species.

### Experimental Example 7: Evaluation of Therapeutic Effect in Wet-AMD Model with CNV and Evaluation of Therapeutic Effect Depending on Concentration

Changes after administering Compound 1-2 at concentrations of 25, 50 and 100 mg/kg for 7 days after induction of the disease without administration of Compound 1-2 before induction of the disease to the wet-AMD mouse model induced in the same manner as in Experimental Example 1 were analyzed. In the same manner as Experimental Example 1, the image of the lesion was obtained and the volume of the lesion was quantified.

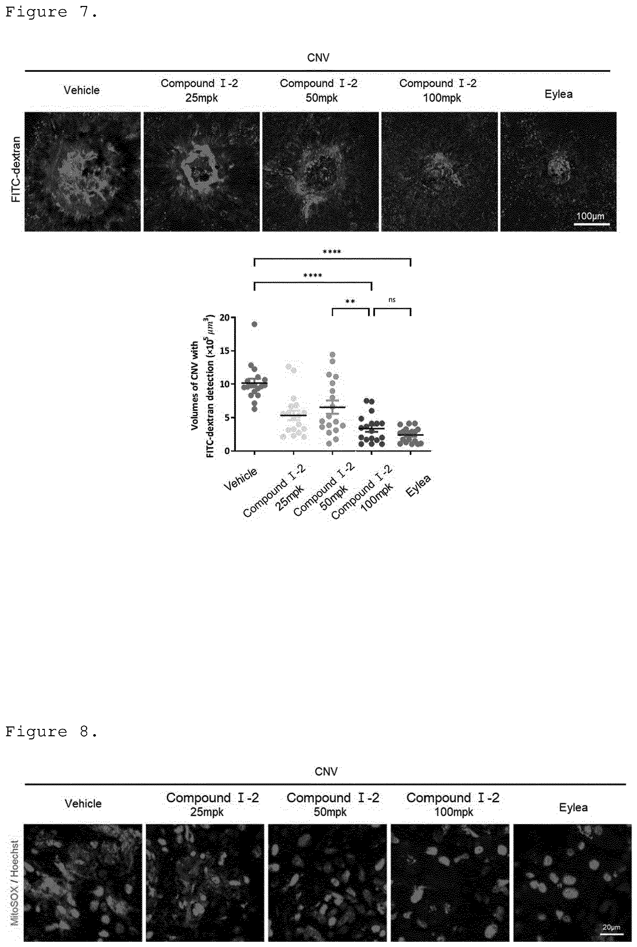

FIG. 7 shows images of the ocular lesions in the wet-AMD animal model and the results of quantitatively measuring the volumes of the lesions, according to Experimental Example 7. Referring to FIG. 7, it can be found through the 3D images and the quantitative reduction in the lesion volume that Compound 1-2 suppressed formation of the lesion even when it was administered after induction of the disease, and the lesion was reduced in a manner dependent on the concentration.

### Experimental Example 8: Analysis of Changes in Mitochondrial Reactive Oxygen Species by Therapeutic Effect in CNV Animal Model (Wet-AMD)

RPE cells were isolated from the same model as in Experimental Example 7 and stained with MitoSOX, and changes in mitochondrial reactive oxygen species in the RPE cells were examined through fluorescence microscopy. Measurement was performed in the same manner as in Experimental Example 2.

FIG. 8 shows the results of analyzing changes in mitochondrial reactive oxygen species in the RPE cells from the wet-AMD animal model according to Experimental Example 8. Referring to FIG. 8, it can be found that Compound 1-2 reduced mitochondrial reactive oxygen species in the wet-AMD model even when it was administered after induction of the disease, and that Compound 1-2 reduced mitochondrial reactive oxygen species in a concentration-dependent manner and exhibited the highest efficacy at 100 mg/kg.

### Experimental Example 9: Construction of Alu-RNA Animal Model (dry-AMD) and Fundus Photography

Male C57BL/6 mice were anesthetized, and a lesion was induced by making a hole in the corneal limbus of the mouse eye and injecting 1.2 µg of Alu-RNA into the subretinal space by a Hamilton syringe. Each of Compound 1-2 and Compound 1-18 was administered orally starting from 6 days before induction of the dry-AMD model, and was administered orally for 7 days after induction of the disease. To evaluate the dry-AMD preventive and therapeutic effects of each of Compound 1-2 and Compound 1-18, the morphology of the lesion was evaluated by fundus photography using TOPCON #TRC-50DX.

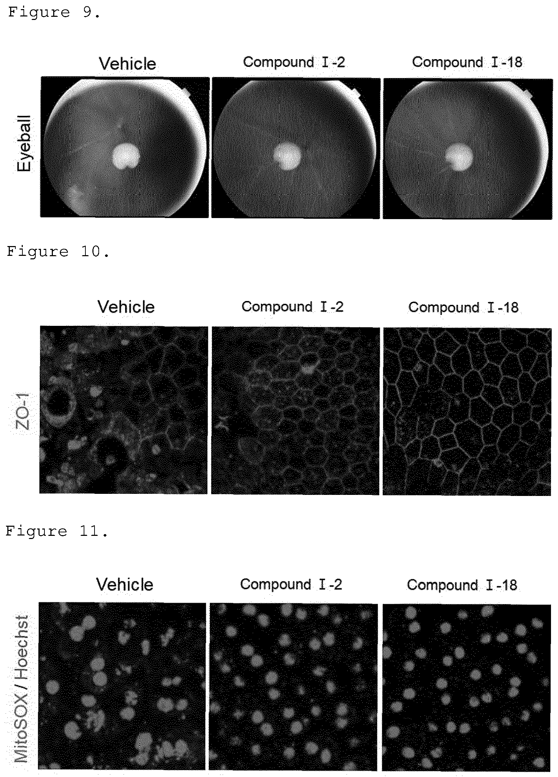

FIG. 9 shows the results of observing the lesion in the eyeball in the dry-AMD animal model according to Experimental Example 9. Referring to FIG. 9, it can be found that the administration of each of Compound 1-2 and Compound 1-18 reduced the lesion in the eyeball in the dry-AMD model.

### Experimental Example 10: Analysis of RPE Cell Structure in Alu-RNA Animal Model (Dry-AMD)

RPE cells were isolated from the same model as in Experimental Example 9 and stained for ZO-1, and changes in the structure of the RPE cells were examined under a fluorescence microscope (Olympus SZ51, Tokyo, Japan). Specifically, immediately after enucleation of the mouse eye, the RPE cytoskeleton was stained for zonula occludens-1 (ZO-1) and imaged with a fluorescence microscope through the Z-stack imaging technique, and changes in the RPE cytoskeleton were imaged.

FIG. 10 shows the results of analyzing changes in the RPE cytoskeleton in the dry-AMD animal model according to Experimental Example 10. Referring to FIG. 10, it can be found that the administration of each of Compound 1-2 and Compound 1-18 restored the damaged RPE cytoskeleton in the dry-AMD model.

### Experimental Example 11: Analysis of Mitochondrial Reactive Oxygen Species in Alu-RNA Animal Model (Dry-AMD)

RPE cells were isolated from the same model as in Experimental Example 9 and stained with MitoSOX, and changes in mitochondrial reactive oxygen species in the RPE cells were examined through fluorescence microscopy. Measurement was performed in the same manner as in Experimental Example 2.

FIG. 11 shows the results of analyzing changes in mitochondrial reactive oxygen species in the RPE cells from the dry-AMD animal model according to Experimental Example 11. Referring to FIG. 11, it can be found that administration of each of Compound 1-2 and Compound 1-18 reduced mitochondrial reactive oxygen species in the RPE cells from the dry-AMD model.

### Experimental Example 12: Analysis of Inflammatory Factors in Alu-RNA Animal Model (Dry-AMD)

RPE cells were isolated from the same model as in Experimental Example 9, and the relative expression level of inflammation-related mRNA was analyzed using real-time PCR. Specifically, Trizol (Invitrogen, Carlsbad, CA, USA) was added to RPE cells which were then disrupted using a homogenizer, and total RNA was extracted from the cells. 100 pmol of oligo Dt17 primer was added to 1 µg of total RNA which was then incubated at 74°C for 10 minutes, and then 30 U of RNAsin (RNase inhibitor), 10 U of AMV-RT (avian myeloblastosis virus-reverse transcriptase), and AMV-RT buffer (Promega, Israel) were added thereto and reacted at 42°C for 2 hours and at 52°C for 1 hour, thereby synthesizing cDNA from the RNA extracted from the liver tissue. cDNA was amplified using the SYBR Premix Ex Taq kit (Takara Bio, Inc.) and the StepOnePlus Real-time PCR system (Applied Biosystems), and then mRNA expression was measured.

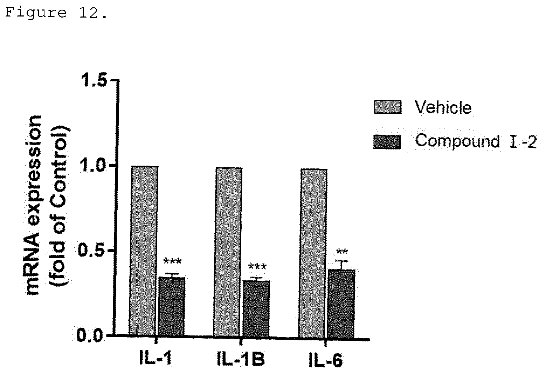

FIG. 12 shows the results of measuring inflammatory factors in the RPE cells in the dry-AMD animal model according to Experimental Example 12. Referring to FIG. 12, it can be found that inflammatory factors in the RPE cells from the dry-AMD model were effectively reduced by administration of Compound 1-2.

Although the present invention has been described with reference to limited embodiments, the present invention is not limited thereto, and it should be understood that various modifications and variations can be made by those skilled in the art to which the present invention pertains, within the scope of the technical idea of the present invention and equivalents to the appended claims.

### Industrial Applicability

The pharmaceutical composition containing a pyrano[2,3-f]chromene derivative compound, a pharmaceutically acceptable salt thereof, or a solvate thereof according to one embodiment of the present invention has an excellent effect of preventing and treating degenerative eye diseases, especially age-related macular degeneration, and thus is industrially applicable.

## Claims

1. A pharmaceutical composition for preventing or treating degenerative eye disease containing a pyrano[2,3-f]chromene derivative compound of the following Formula I, a pharmaceutically acceptable salt thereof, or a solvate thereof: 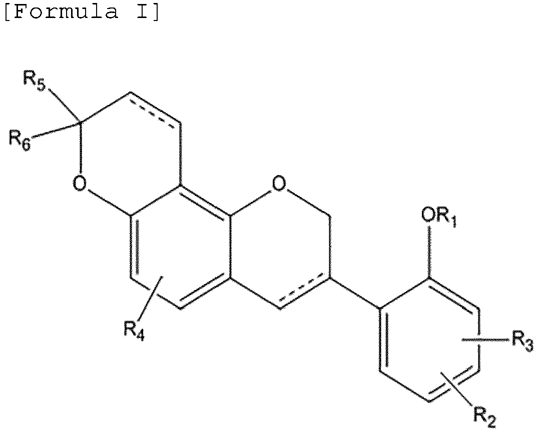 wherein:
the dotted line represents an optional double bond;
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

2. The pharmaceutical composition according to claim 1,
wherein
R₁ is a hydrogen atom, a methyl group, or an ethyl group,
R₂ is a methyl group, an ethyl group, a n-propyl group, an iso-propyl group, a n-butyl group, an iso-butyl group, a tert-butyl group, a methoxy group, an ethoxy group, a n-propoxy group, an iso-propoxy group, a n-butoxy group, an iso-butoxy group, a tert-butoxy group, or a hydroxyl group,
R₃ and R₄ are each a hydrogen atom, and
R₅ and R₆ are each independently a methyl group.

3. The pharmaceutical composition according to claim 1, wherein the pyrano[2,3-f]chromene derivative compound of Formula I comprises a compound of the following Formula I-1: 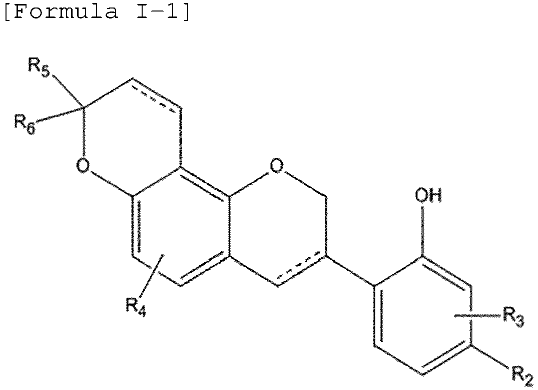 wherein:
the dotted line represents an optional double bond;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

4. The pharmaceutical composition according to claim 1, wherein the pyrano[2,3-f]chromene derivative compound of Formula I comprises a compound of the following Formula II: 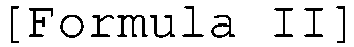 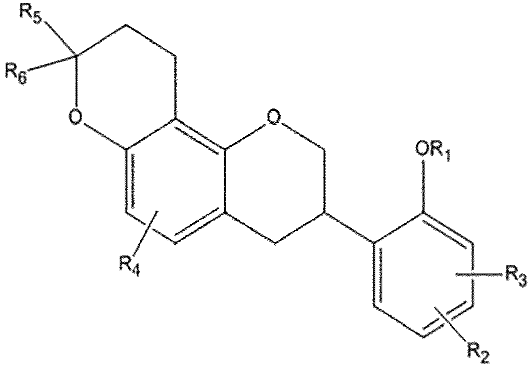 wherein:
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

5. The pharmaceutical composition according to claim 1, wherein the pyrano[2,3-f]chromene derivative compound of Formula I comprises a compound of the following Formula II-1: 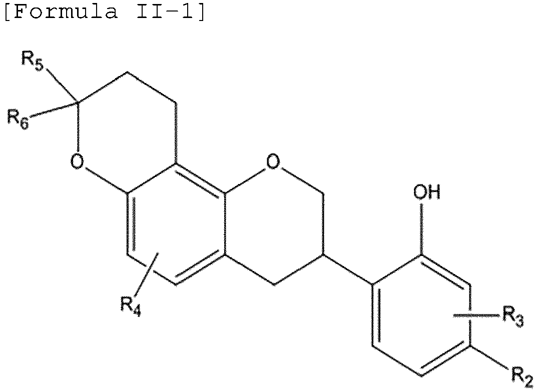 wherein:
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

6. The pharmaceutical composition according to claim 5, wherein the pyrano[2,3-f]chromene derivative compound of Formula I comprises at least one of the following compounds:

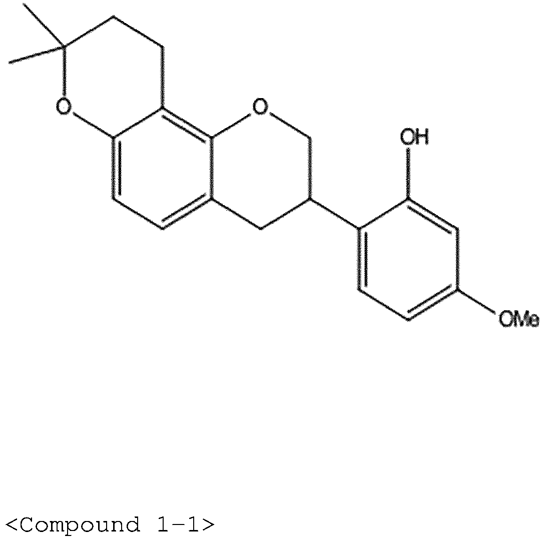

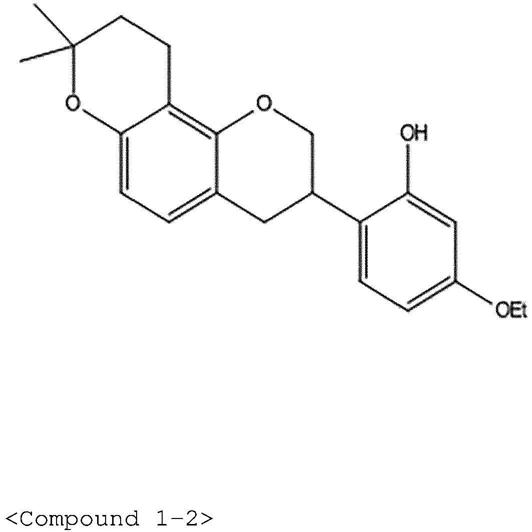

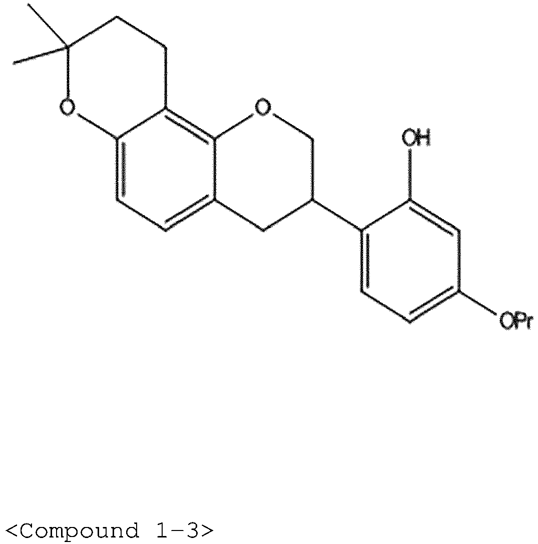

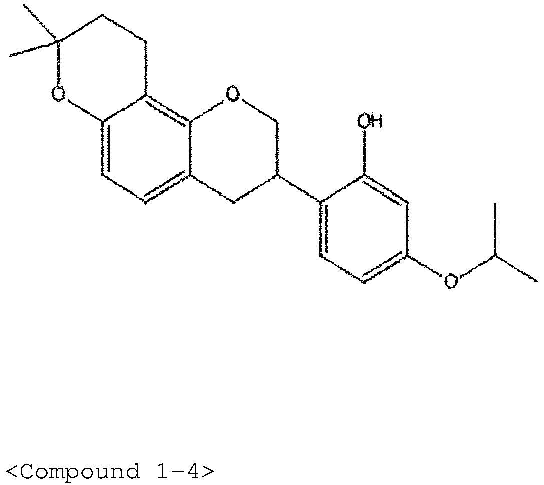

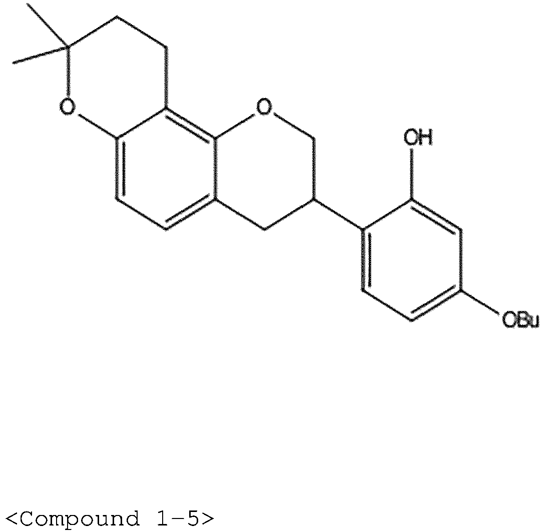

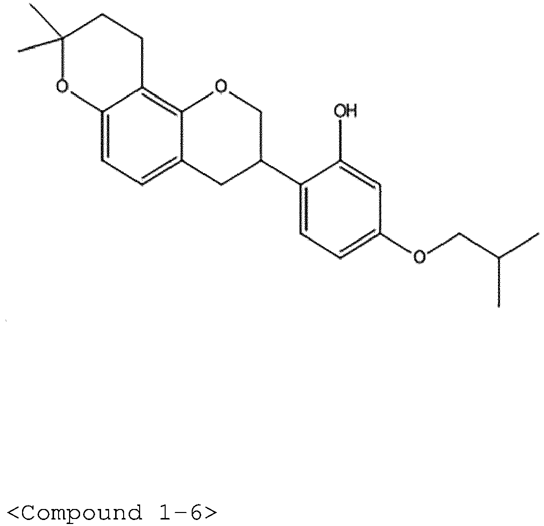

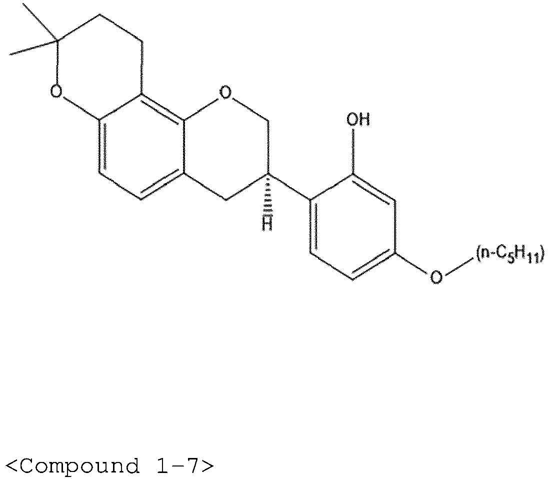

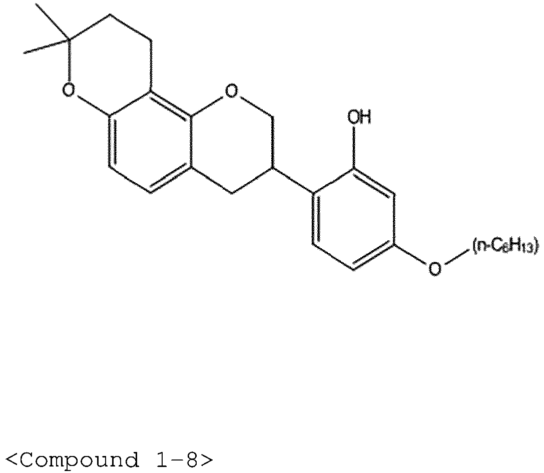

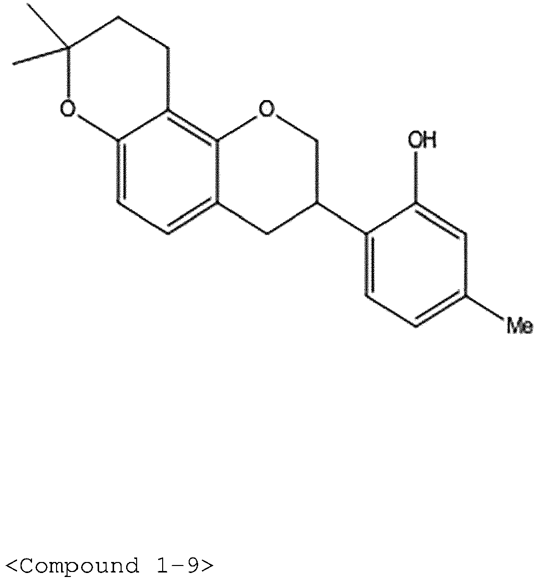

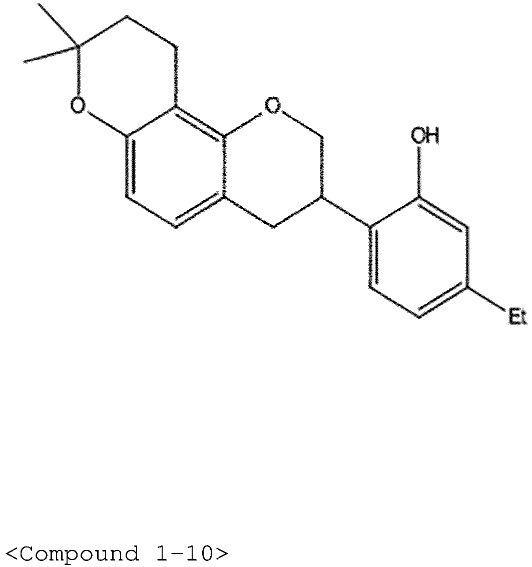

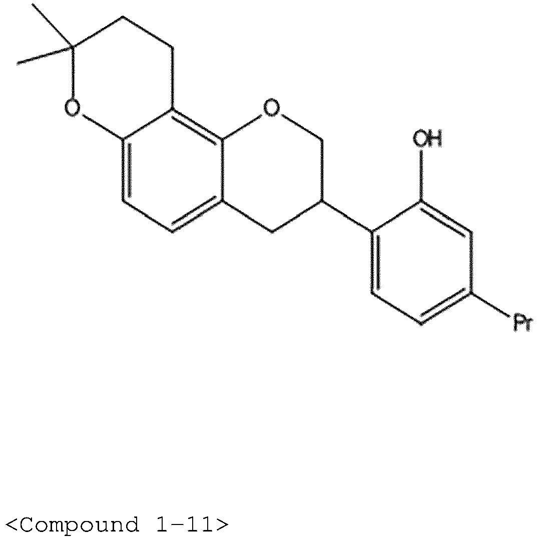

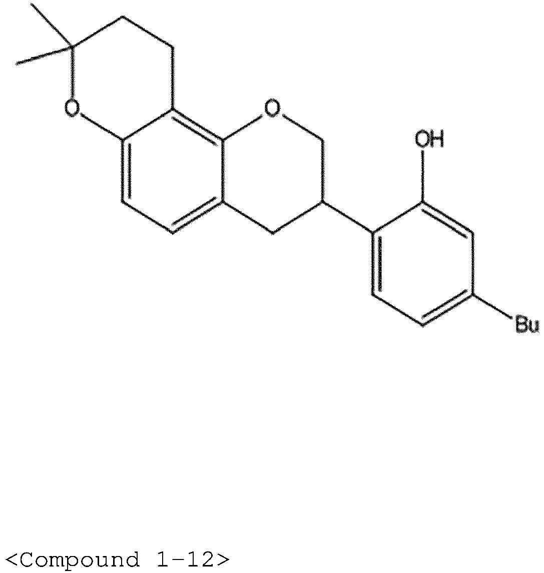

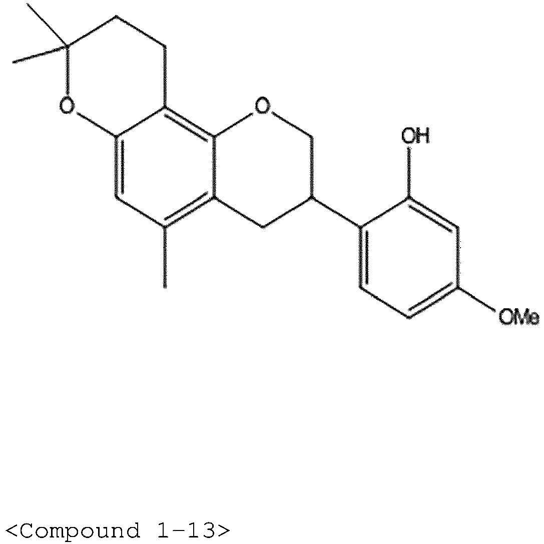

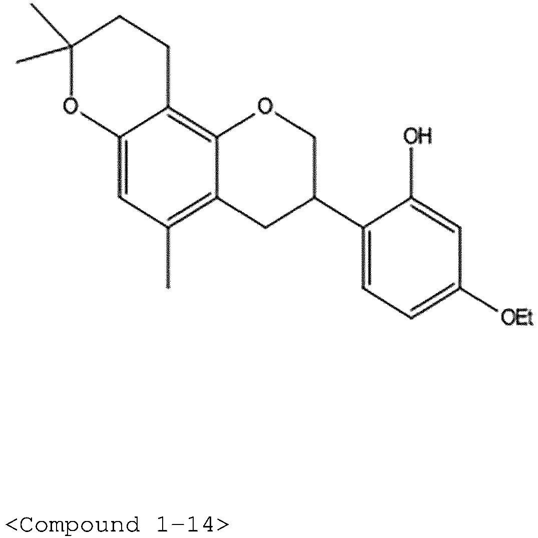

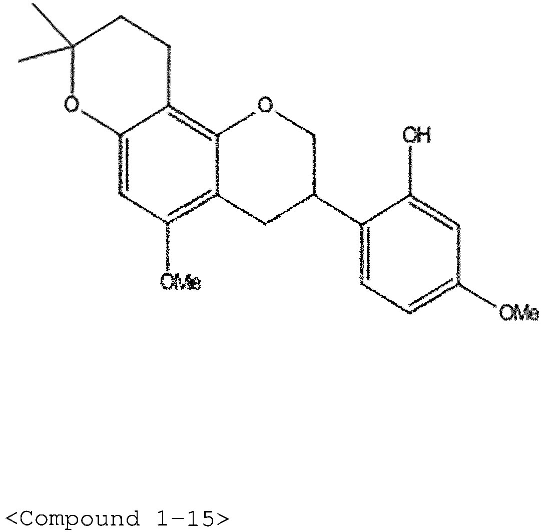

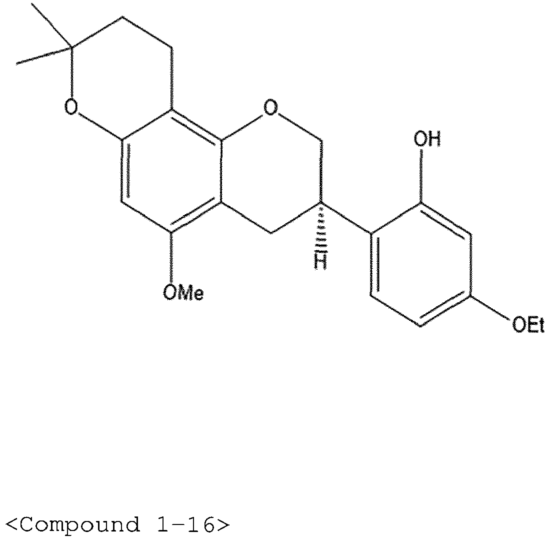

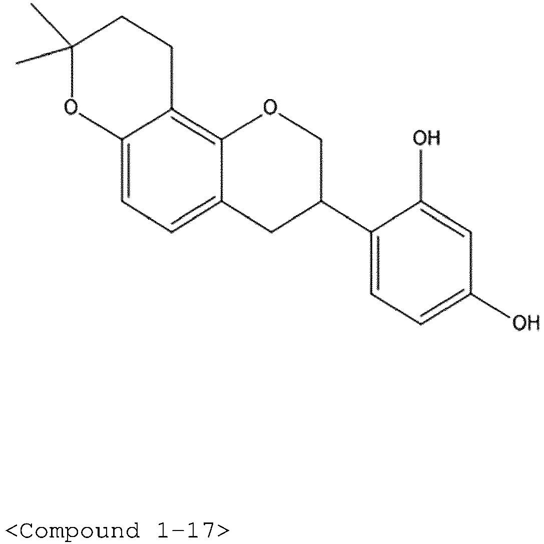

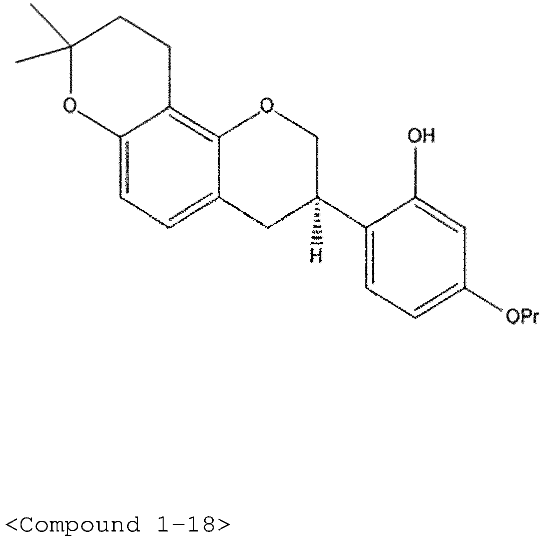

7. The pharmaceutical composition according to claim 1, wherein the pyrano[2,3-f]chromene derivative compound of Formula I comprises a compound of the following Formula III: 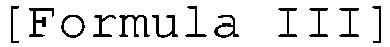 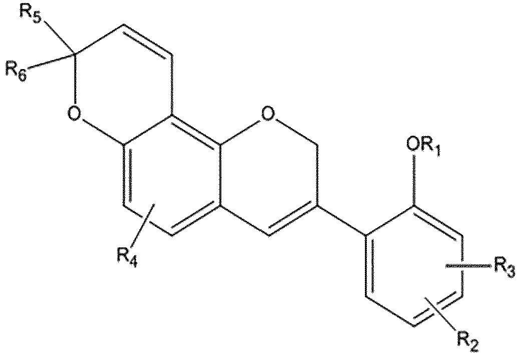 wherein:
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

8. The pharmaceutical composition according to claim 1, wherein the pyrano[2,3-f]chromene derivative compound of Formula I comprises a compound of the following Formula III-1: 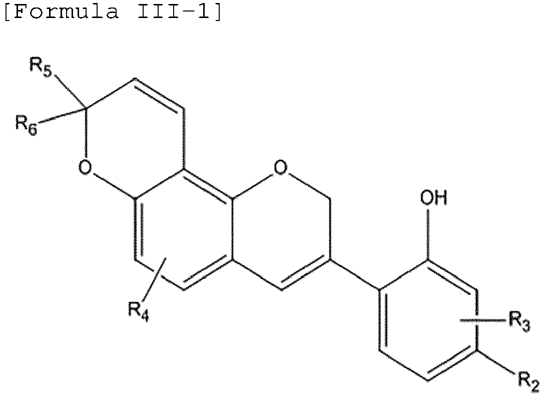 wherein:
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

9. The pharmaceutical composition according to claim 8, wherein the pyrano[2,3-f]chromene derivative compound of Formula I comprises at least one of the following compounds:

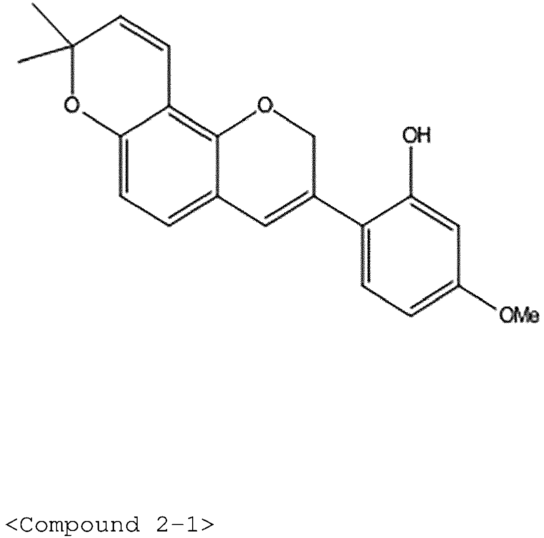

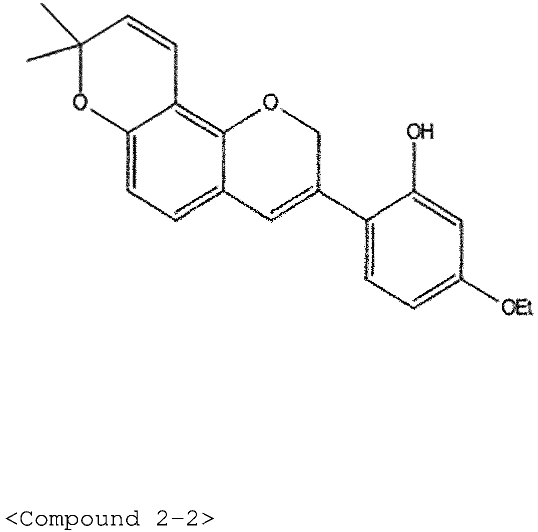

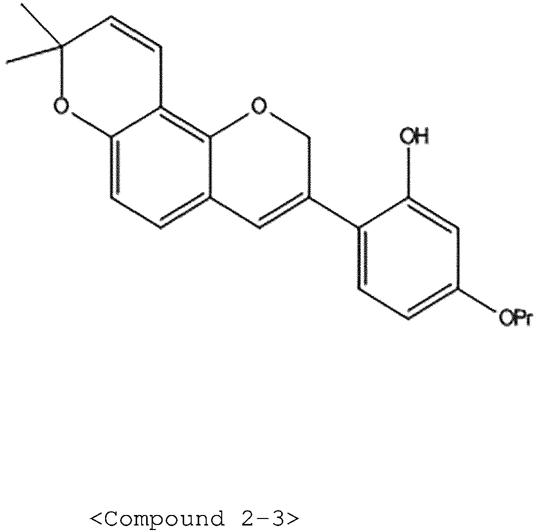

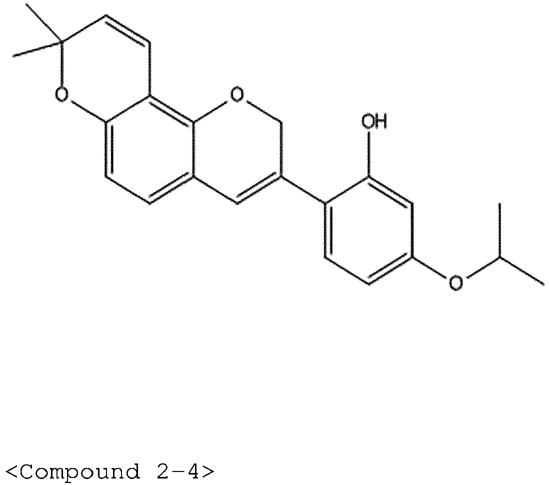

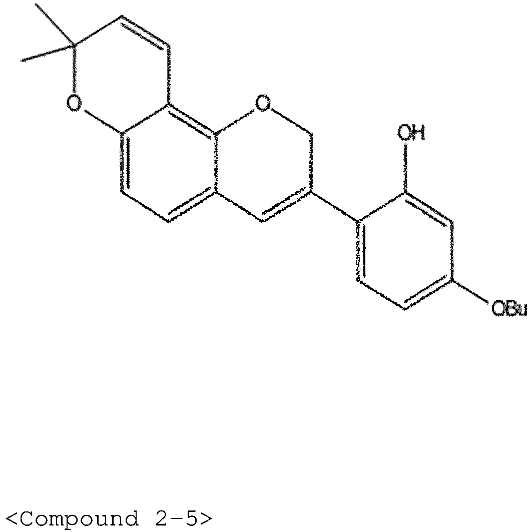

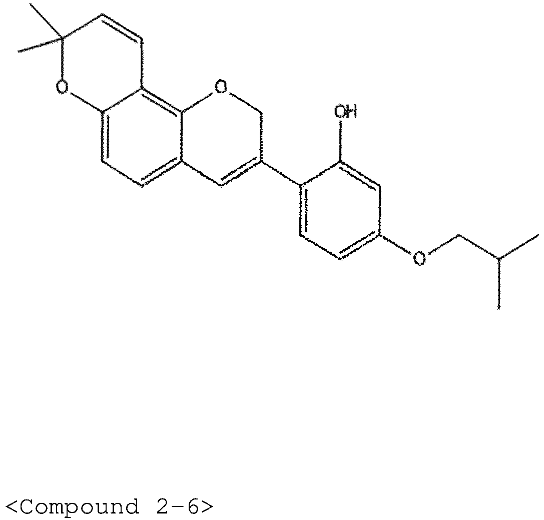

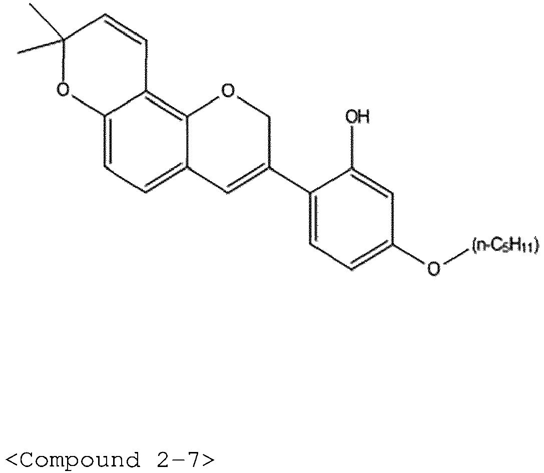

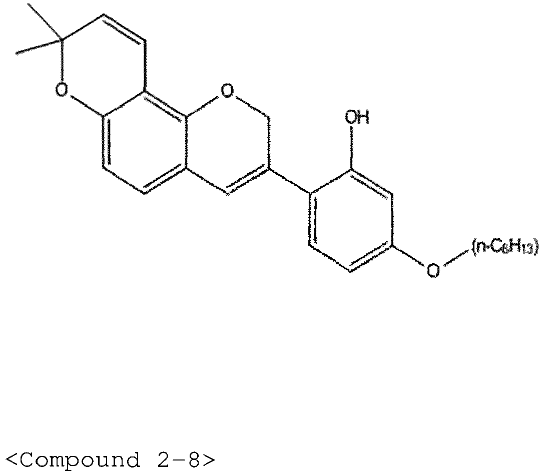

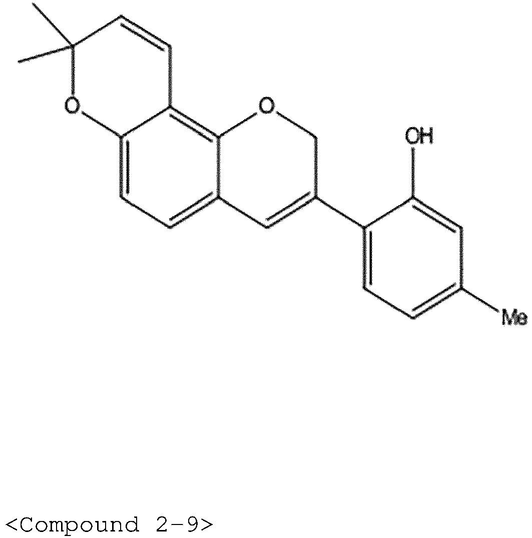

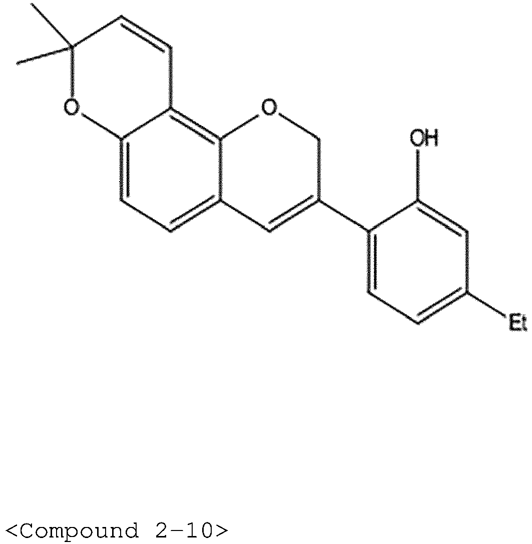

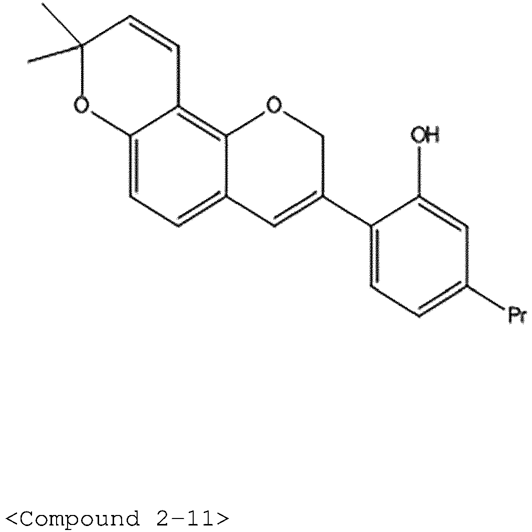

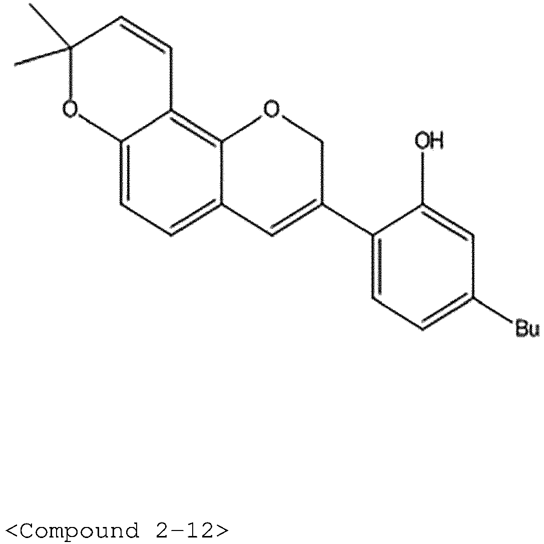

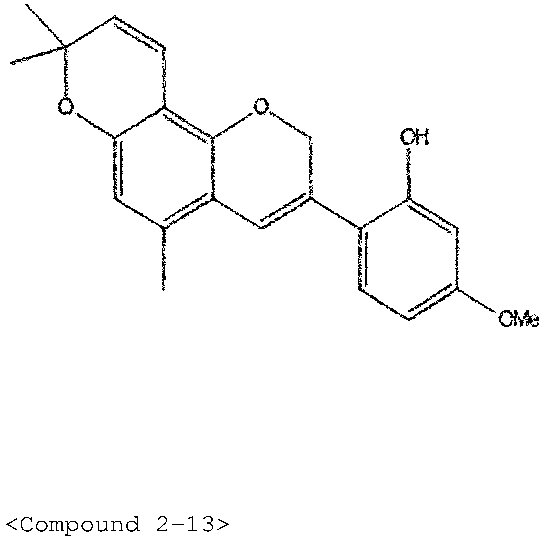

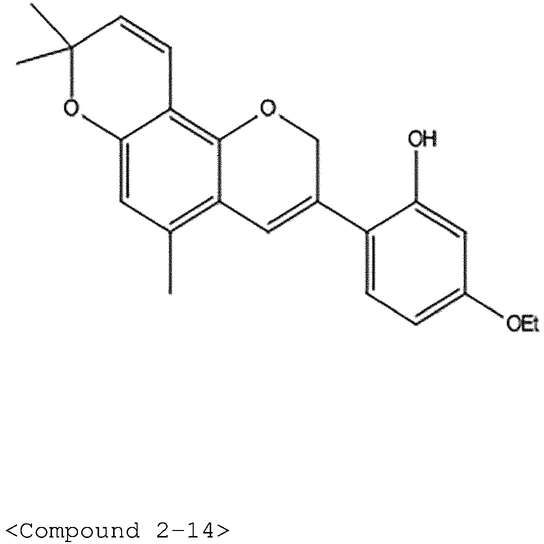

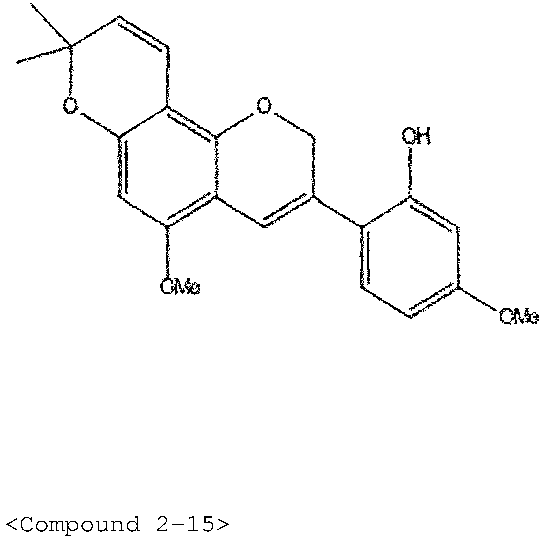

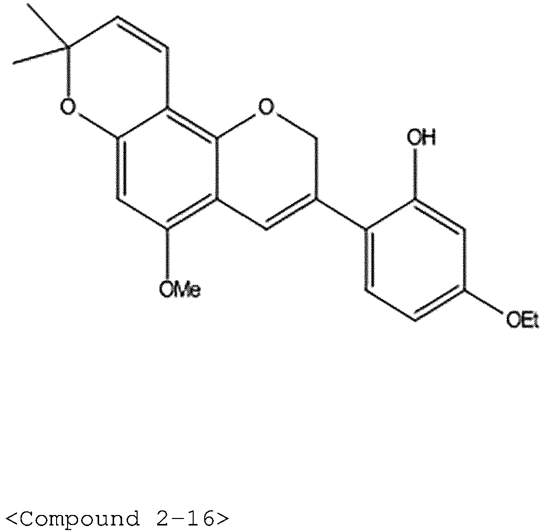

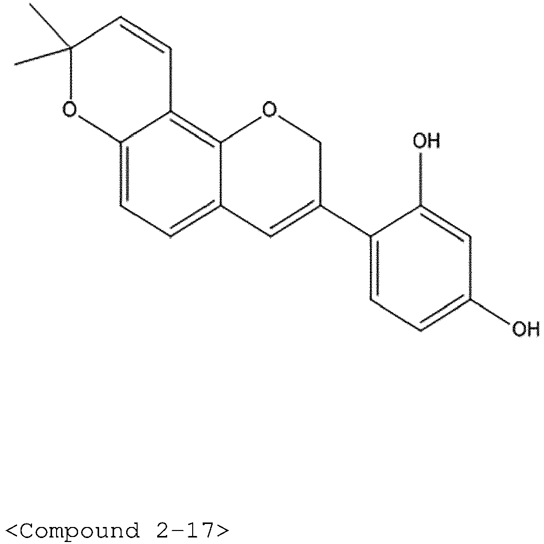

10. The pharmaceutical composition according to claim 1, wherein the degenerative eye disease is one or more selected from macular degeneration, diabetic retinopathy, glaucoma, and cataracts.

11. The pharmaceutical composition according to claim 10, wherein the macular degeneration is wet macular degeneration or dry macular degeneration.

12. A method for preventing or treating degenerative eye disease comprising a step of administering, to a subject suspected of having degenerative eye disease, a composition containing a pyrano[2,3-f]chromene derivative compound of the following Formula I, a pharmaceutically acceptable salt thereof, or a solvate thereof: 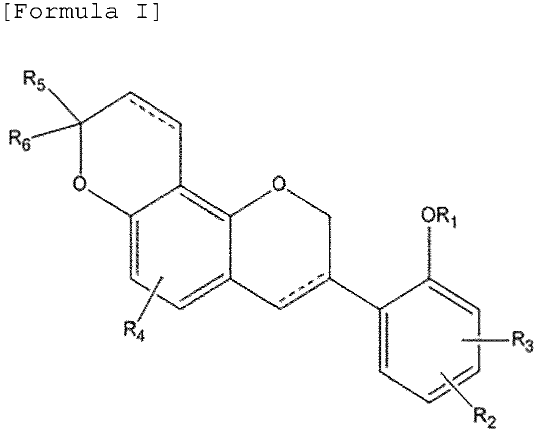 wherein:
the dotted line represents an optional double bond;
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.

13. Use of a composition containing a pyrano[2,3-f]chromene derivative compound of the following Formula I, a pharmaceutically acceptable salt thereof, or a solvate thereof for preventing or treating degenerative eye disease: 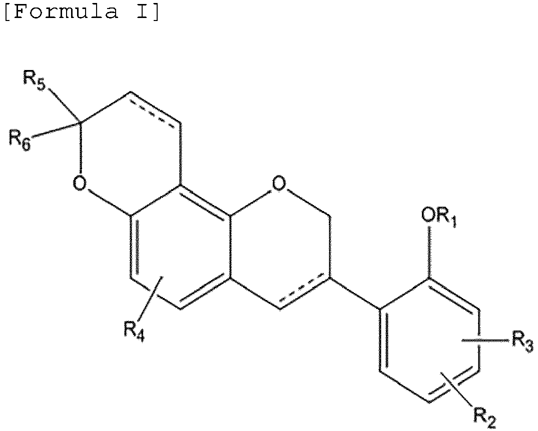 wherein:
the dotted line represents an optional double bond;
R₁ is any one of a hydrogen atom and a substituted or unsubstituted linear or branched C₁-C₆ alkyl group;
R₂ is any one of a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, and a hydroxyl group;
R₃ is any one of a hydrogen atom, a substituted or unsubstituted linear or branched C₁-C₆ alkyl group, a substituted or unsubstituted linear or branched C₁-C₆ alkoxy group, a substituted or unsubstituted linear or branched C₃-C₆ allyloxy group, a substituted or unsubstituted C₆-C₁₂ aryloxy group, a substituted or unsubstituted linear or branched C₁-C₄ thioalkyl group, and a halogen atom;
R₄ is a hydrogen atom, methyl, ethyl, methoxy, or ethoxy; and
R₅ and R₆ are each independently any one of a hydrogen atom and a C₁-C₆ alkyl group,
wherein the substituent of each of the substituted alkyl group, the substituted alkoxy group, the substituted allyloxy group, the substituted aryloxy group, and the substituted thioalkyl group is any one of a halogen atom, a linear or branched C₁-C₅ alkyl group, a linear or branched C₁-C₅ alkoxy group, and a linear or branched C₁-C₃ thioalkyl group.
